(19) Europäisches Patentamt
European Patent Office
Office européen des brevets

(11) EP 0 987 260 B1

(12) EUROPÄISCHE PATENTSCHRIFT

(45) Veröffentlichungstag und Bekanntmachung des Hinweises auf die Patenterteilung:
**03.03.2004 Patentblatt 2004/10**

(51) Int Cl.7: **C07D 311/78**, C07D 495/10, G02B 5/23, C08K 5/15

(21) Anmeldenummer: **99109857.5**

(22) Anmeldetag: **19.05.1999**

# (54) Photochrome Spirofluorenopyrane

Photochromic spirofluorenopyrans

Spirofluorénopyrannes photochromes

(84) Benannte Vertragsstaaten:
**DE FR GB IT**

(30) Priorität: **29.05.1998 DE 19824278**
**25.01.1999 DE 19902771**

(43) Veröffentlichungstag der Anmeldung:
**22.03.2000 Patentblatt 2000/12**

(73) Patentinhaber: **Rodenstock GmbH**
**80469 München (DE)**

(72) Erfinder:
- **Mann, Claudia, Dr.**
  **80634 München (DE)**
- **Melzig, Manfred, Dr.**
  **82234 Wessling (DE)**
- **Weigand, Udo, Dr.**
  **80638 München (DE)**

(74) Vertreter: **Meissner, Bolte & Partner**
**Anwaltssozietät GbR**
**Postfach 86 06 24**
**81633 München (DE)**

(56) Entgegenhaltungen:
**WO-A-96/14596**
**WO-A-97/48762**
**WO-A-97/48993**
**WO-A-99/15518**

EP 0 987 260 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft photochrome Spirofluorenopyrane sowie ihre Verwendung. Es handelt sich bei den erfindungsgemäßen Verbindungen um photochrome Pyran-Verbindungen, die vom 9H-Fluoren abgeleitet sind, wobei das Kohlenstoffatom in 9-Position einem weiteren Ringsystem angehört und somit eine Spiro-Verknüpfungsstelle bildet.

Seit langern sind verschiedene Farbstoffklassen bekannt, die bei Bestrahlung mit Licht bestimmter Wellenlängen, insbesondere Sonnenstrahlen, reversibel ihre Farbe wechseln. Dies geht darauf zurück, daß diese Farbstoffmoleküle durch Lichtenergie in einen angeregten Zustand übergehen, den sie bei Unterbrechung der Energiezufuhr wieder verlassen und in ihren Ausgangszustand zurückkehren. Zu diesen photochromen Farbstoffen gehören verschiedene Pyransysteme, die im Stand der Technik mit unterschiedlichen Grundsystemen und Substituenten bereits beschrieben wurden.

Stand der Technik

Pyrane, speziell Naphthopyrane und von diesen abgeleitete größere Ringsysteme, sind derzeit die am meisten bearbeitete Klasse photochromer Verbindungen. Obwohl bereits im Jahr 1966 erstmals zum Patent angemeldet (US 3.567.605), konnten erst in den 90er Jahren Verbindungen entwickelt werden, die für den Einsatz in Brillengläsern geeignet erschienen. Eine geeignete Verbindungsklasse von Pyranen sind zum Beispiel die 2,2-Diaryl-2H-naphtho [1,2-b]pyrane oder die 3,3-Diaryl-3H-naphtho[2,1-b]pyrane, die in angeregter Form verschiedene Färbungen, wie Gelb, Orange oder Rotorange, zeigen. Als weitere Verbindungsklasse photochromer Verbindungen sind die indenoannellierten Naphthopyrane von Interesse, die aufgrund ihres größeren Ringsystems längerwellig absorbieren. Diese können entweder von den 2H-Naphtho[1,2-b]pyranen oder den 3H-Naphtho[2,1-b]pyranen abgeleitete Systeme sein, wobei sie durch Annellierung an der f-Seite des Naphthalinteils aus den jeweiligen Naphthopyransystemen hervorgehen.

Mehrere Druckschriften beschäftigen sich mit von den 2H-Naphtho[1,2-b]pyranen abgeleiteten indenoannellierten Naphthopyranen:

Die WO 97/48762, die WO 97/48993 und die US 5.723.072 beschreiben Naphthopyranverbindungen mit einer substituierten oder unsubstituierten Indenogruppe, deren 2,1-Positionen mit der f-Seite des Naphthalinteils eines 2H-Naphtho[1,2-b]pyrans annelliert sind, wobei der Pyranring spezielle Substituenten besitzt. In der WO 97/48993 und der US 5.723.072 kann zusätzlich an diesem Grundsystem ein un-, mono- oder disubstituierter heterozyklischer Ring an der g-, h-, i-, n-, o- oder p-Seite des Indenonaphthopyrans annelliert sein. Es werden demzufolge Indeno[2,1-f] naphtho[1,2-b]pyrane mit einer sehr großen Variationsbreite an möglichen Substituenten offenbart, wobei in diesen Druckschriften allerdings keine Ringbildung zwischen den Substituenten $R_1$ und $R_2$ am Kohlenstoffatom Nr. 13 unter Ausbildung eines Spiro-Kohlenstoffatoms beschrieben ist. Eine Spiro-Verbindung wird überhaupt nur direkt am Pyranring bei den Substituenten B und B' offenbart; erfindungsgemäß werden demgegenüber andere Spiro-Verbindungen beansprucht.

Auch die WO 96/14596, die PCT/DE 98/02820, die US 5.645.767, die WO 98/32037 und die US 5.698.141 beschreiben vom 2H-Naphtho[1,2-b]pyran abgeleitete photochrome indenoannellierte Naphthopyran-Farbstoffe, die sie enthaltenden Zusammensetzungen sowie ein Verfahren zu ihrer Herstellung. In der US 5.698.141 kann zusätzlich an diesem Grundsystem ein un-, mono- oder disubstituierter heterozyklischer Ring an der g-, h-, i-, n-, o- oder p-Seite des Indenonaphthopyrans annelliert sein. Von der jeweils sehr umfangreichen Substituentenliste sind auch ganz spezielle Spiro-Verbindungen umfaßt, und zwar solche Systeme mit einer spiroheterozyklischen Gruppe, worin einschließlich des Spiroatoms an 13-Position des Grundsystems ein 5- bis 8-gliedriger Ring, der stets 2 Sauerstoffatome enthält, vorhanden ist.

Die verschiedenen bekannten photochromen Farbstoffe sind jedoch mit Nachteilen verbunden, die bei deren Verwendung in Sonnenschutzgläsern den Tragekomfort des Brillen trägers wesentlich beeinträchtigen. Die bekannten Farbstoffe weisen eine nicht ausreichend langwellige Absorption im angeregten wie im nicht angeregten Zustand auf. Es liegt eine zu hohe Temperaturempfindlichkeit der Eindunkelung vor, wobei gleichzeitig häufig eine zu langsame Aufhellung eintritt. Darüber hinaus besitzen die beschriebenen Farbstoffe oft eine ungenügende Lebensdauer und erlauben damit nur eine geringe Haltbarkeit der Sonnenschutzgläser. Letzteres macht sich in schnell nachlassender Leistung und/oder starker Vergilbung bemerkbar.

Im Stand der Technik findet der Fachmann zudem kaum Hinweise, an welchen Stellen und mit welchen Substituenten ein Pyran zu substituieren ist, um ein bestimmtes langwelliges Absorptionsmaximum zu erreichen.

In den Fällen, in denen bestimmte Substitutionsmuster ein Absorptionsmaximum festlegen, sind damit auch die kinetischen Eigenschaften festgelegt, wie Eindunkelungs- und Aufhellgeschwindigkeit, Temperaturabhängigkeit u. a. Eine beliebige Beeinflussung der Kinetik in beide Richtungen, schneller oder langsamer, und praktisch ohne Einfluß auf das Absorptionsmaximum, war bisher nicht möglich.

Hintergrund der Erfindung

Der vorliegenden Erfindung liegt daher die Aufgabe zugrunde, eine neue Klasse photochromer Verbindung bereitzustellen, die deutlich verbesserte Eigenschaften gegenüber den im Stand der Technik beschriebenen Strukturen besitzen. Darüber hinaus soll es dem Fachmann ermöglicht werden, durch entsprechende Auswahlregeln für den jeweiligen Verwendungszweck maßgeschneiderte Eigenschaften, wie Absorptionsmaximum (Farbe), Aufhellgeschwindigkeit u.a. zu erzielen.

Die oben erwähnte Aufgabe der Erfindung wird gelöst durch die photochromen Spirofluorenopyrane der Formel (I):

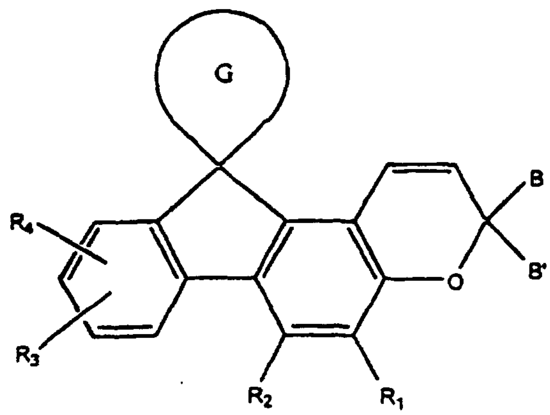

(I)

worin

$R_1$ ein Substituent, ausgewählt aus der Gruppe A, bestehend aus ($C_1$-$C_6$)-Alkyl, ($C_1$-$C_6$)-Alkoxy, Phenyl, Brom, Chlor und Fluor, ist;

$R_2$, $R_3$ und $R_4$ unabhängig voneinander gleich oder verschieden sind und einen Substituenten, ausgewählt aus der Gruppe A', bestehend aus Wasserstoff und den Substituenten der Gruppe A, darstellen;

oder

($R_1$ zusammen mit $R_2$) und/oder ($R_3$ zusammen mit $R_4$) unabhängig voneinander einen unsubstituierten, mono- oder disubstituierten Benzo- oder Pyridoring darstellen, dessen Substituenten aus der Gruppe A ausgewählt sind;

G unter Einrechnung des Spiro-Kohlenstoffatoms einen 5- bis 8-gliedrigen Ring darstellt, an den mindestens ein aromatisches oder heteroaromatisches Ringsystem annelliert ist, wobei das oder die Ringsysteme ausgewählt sind aus der Gruppe E, bestehend aus Benzol, Naphthalin, Phenanthren, Pyridin, Chinolin, Furan, Thiophen, Pyrrol, Benzofuran, Benzothiophen, Indol und Carbazol, die wiederum einen oder zwei Substituenten, ausgewählt aus der Gruppe A, aufweisen können;

und

B und B' unabhängig voneinander aus den folgenden Gruppen a), b), c) oder d) ausgewählt sind, mit

a) Phenyl- oder Naphthyl, die un-, mono-, di- und trisubstituiert sind; oder
b) den Heterozyklen Pyridyl, Furanyl, Benzofuranyl, Thienyl, Benzothienyl und Julolidinyl, die un-, mono- oder disubstituiert sind;
wobei der oder die Substituenten der Aryl- oder Heteroarylgruppen in a) und b) aus einer Gruppe F, bestehend aus Hydroxy, Amino, Mono-($C_1$-$C_6$-alkylamino, Di-($C_1$-$C_6$)-alkylamino, am Phenylring un-, mono-

oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, Carbazolyl, un-, mono- und disubstituiertem Pyrryl, $(C_1-C_6)$-Alkyl, $(C_1-C_6)$-Alkoxy, Brom, Chlor und Fluor ausgewählt sind, wobei der oder die Substituenten an den Aromaten und Heteroaromaten aus einer Gruppe, bestehend aus $(C_1-C_6)$-Alkyl, $(C_1-C_6)$-Alkoxy, Brom, Chlor und Fluor, ausgewählt sind; oder

c) den Gruppen mit den folgenden Strukturbildern:

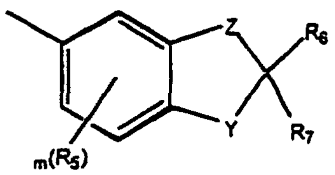
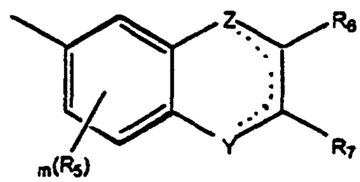
,

worin

Y und Z unabhängig voneinander aus der Gruppe O, S, CH, $CH_2$, $NR^N$ und die Stickstoffsubstituenten $R^N$ aus der Gruppe, bestehend aus $(C_1-C_6)$-Alkyl, $(C_1-C_6)$-Acyl, Phenyl und Wasserstoff, ausgewählt sind und die Substituenten $R_5$ aus der Gruppe A und Hydroxy ausgewählt sind, wobei m = 0, 1 oder 2 ist, und $R_6$ und $R_7$ unabhängig voneinander Wasserstoff und/oder $(C_1-C_6)$-Alkyl sind; oder

d) B und B' zusammen un-, mono- oder disubstituiertes Fluoren-9-yliden oder einen gesättigten Kohlenwasserstoff darstellen, der $C_3$-$C_{12}$-spiro-monozyklisch, $C_7$-$C_{12}$-spiro-bizyklisch und/oder $C_7$-$C_{12}$-spiro-trizyklisch ist, wobei die Fluorensubstituenten aus der Gruppe A ausgewählt sind.

Demzufolge stellt das Ringsystem G unter Hinzunahme des Spiro-Kohlenstoffatoms einen 5-gliedrigen bis 8-gliedrigen Ring dar. An diesen Ring ist mindestens ein aromatisches oder heteroaromatisches Ringsystem annelliert. Das oder die annellierten Ringsysteme können erfindungsgemäß unabhängig voneinander ausgewählt sein aus der Gruppe, bestehend aus Benzol, Naphthalin, Phenanthren, Pyridin, Chinolin, Furan, Thiophen, Pyrrol, Benzofuran, Benzothiophen, Indol und Carbazol. Selbstverständlich können die Ringsysteme sowohl unals auch mono- oder disubstituiert sein, wobei der oder die Substituenten aus der oben definierten Gruppe A ausgewählt sind.

In der vorliegenden Erfindung soll unter $C_3$-$C_{12}$-spiro-monozyklisch ein dem Fachmann bekannter 3-gliedriger bis 12-gliedriger Ring verstanden werden. Auch $C_7$-$C_{12}$-spiro-bizyklische Systeme, die erfindungsgemäß vorhanden sein können, gehören zum Wissen des Fachmanns. Beispielhaft seien Norbornan, Norbornen, 2,5-Norbornadien, Norcaran und Pinan genannt. Ein in der Erfindung verwendbares bekanntes spiro-trizyklisches System ist beispielsweise das Adamantan.

Gegenstand der Erfindung sind auch photochrome Spirofluorenopyrane der Formel (II):

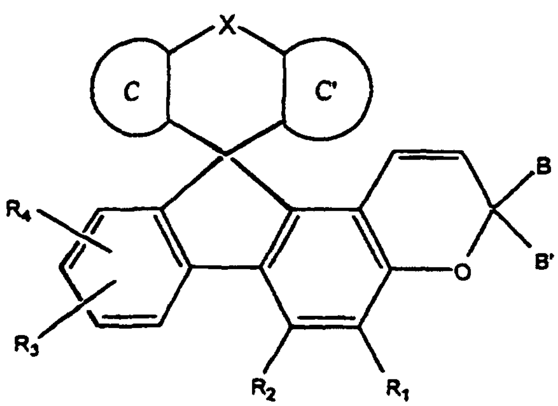
,

(II)

worin $R_1$ bis $R_4$, B und B' wie oben definiert sind;

X entweder eine Einfachbindung ist, oder ausgewählt ist aus der Gruppe, bestehend aus

-O-, -S-, $-(CR_2)_n-$ mit n = 1, 2 oder 3,
-D=D'- mit D, D' = N, CR oder
-L-L'- mit L, L' = O, S, NR, CHR oder $CR_2$,
wobei R = H, $(C_1-C_6)$-Alkyl oder Phenyl und L und L' beide nicht gleichzeitig O oder S sein können;

und
C und C' unabhängig voneinander aus der oben definierten Gruppe E ausgewählt sind, die jeweils einen oder zwei Substituenten, ausgewählt aus der Gruppe A aufweisen können.

Wenn X in der vorliegenden Erfindungsbeschreibung als "Einfachbindung" bezeichnet wird, so bedeutet dies, daß zwischen den beispielsweise zu verknüpfenden Ringsystemen C und C' eine unmittelbare Verknüpfung vorhanden ist, d.h. kein weiteres Atom als Verbrückung dienen soll.

Nach einer weiteren bevorzugten Ausführungsform der Erfindung stellt X eine Einfachbindung dar, wobei die beiden vorhandenen Ringsysteme C und C' durch eine weitere Verknüpfung in ortho,ortho'-Stellung zur ersten Verknüpfung verbrückt sind. Eine derartige Verbrückung umfaßt jede dem Fachmann bekannte Möglichkeit, die beiden Ringsysteme C und C' zu verbinden, wie zum Beispiel durch Heteroatome, wie Sauerstoff oder Schwefel, gesättigte oder ungesättigte $C_2$- bis $C_5$-Kohlenstoffketten oder dergleichen. Beispielsweise kann die weitere Verknüpfung in ortho, ortho'-Stellung zu einer 4,5-Phenanthryl-Spiroverbindung führen, wobei die erfindungsgemäßen Spiro-Verbindungen dann die folgende allgemeine Struktur (III) aufweisen können:

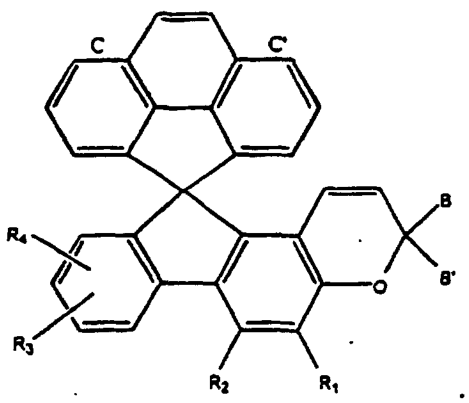

(III)

Es kann auch von Vorteil sein, wenn in den erfindungsgemäßen Spiro-Verbindungen B und B' unabhängig voneinander aus der Gruppe a) oder d) ausgewählt sind. Besonders bevorzugt stellen B und B' unabhängig voneinander un- oder monosubstituiertes Phenyl oder Naphthyl dar, wobei der Substituent jeweils aus der oben definierten Gruppe F ausgewählt ist. In den erfindungsgemäßen photochromen Spiro-Verbindungen können B und B' auch die gleichen Substituenten sein.

Nach einer weiteren bevorzugten Ausführungsform der Erfindung sind C und C' unabhängig voneinander un- oder monosubstituiertes Phenyl oder Naphthyl, wobei der Substituent jeweils aus der Gruppe A ausgewählt ist. Auch können C und C' die gleichen Substituenten darstellen.

Besonders vorteilhafte Eigenschaften besitzen die folgenden erfindungsgemäßen photochromen Spirofluorenopyrane:

1) Spiro-9-fluoren-13'-[3,3-diphenyl-6-methoxy-indeno[2,1-f]naphtho[1,2-b]pyran];
2) Spiro-9-fluoren-13'-[6-methoxy-3-(4-methoxyphenyl)-3-phenyl-indeno[2,1-f]-naphtho[1,2-b]pyran];
3) Spiro-9-xanthen-13'-[6-methoxy-3-(4-methoxyphenyl)-3-phenyl-indeno[2,1-f]-naphtho[1,2-b]pyran];
4) Spiro-9-fluoren-13'-[3,3-bis(4-methoxyphenyl)-6-methoxy-indeno[2,1-f]naphtho-[1,2-b]pyran];
5) Spiro-9-xanthen-13'-[3,3-bis(4-methoxyphenyl)-6-methoxy-indeno[2,1-f]-naphtho[1,2-b]pyran];
6) Spiro-9-(9,10-dihydroanthracen)-13'-[3,3-bis(4-methoxyphenyl)-6-methoxy-indeno[2,1-f]naphtho[1,2-b]pyran];
7) Spiro-9-fluoren-13'-{6-memoxy-3-[4-(N-morpholinyl)phenyl]-3-phenyl-indeno[2,1-f]-naphtho[1,2-b]pyran};
8) Spiro-9-fluoren-13'-[3-(4-dimethylaminophenyl)-6-methoxy-3-phenyl-indeno[2,1-f]-naphtho[1,2-b]pyran];

9) Spiro-9-fluoren-13'-[3-(4-diphenylaminophenyl)-6-methoxy-3-phenyl-indeno[2,1-f]-naphtho[1,2-b]pyran];
10) Spiro-9-fluoren-13'-[3,3-bis(4-methoxyphenyl)-indeno[2,1-f]naphtho[1,2-b]pyran];
11) Spiro-9-fluoren-13'-{3-[4-(N-morpholinyl)phenyl]-3-phenyl-indeno[2,1-f]naphtho-[1,2-b]pyran};
12) Spiro-9-fluoren-11'-[3,3-bis(4-methoxyphenyl)-5-brom-fluoreno[2,1-b]pyran] oder
13) Spiro-9-fluoren-13'-[3,3-bis(4-methoxyphenyl)-5-brom-benzo[1]fluoreno[2,1-b]pyran].

Gegenstand der Erfindung ist ferner die Verwendung eines oder mehrerer der photochromen Spirofluorenopyrane in Kunststoffen aller Art, insbesondere für ophthalmische Zwecke. Beispielsweise können die erfindungsgemäßen photochromen Farbstoffe in Linsen, Gläsern für Brillen aller Art, wie beispielsweise Skibrillen, Sonnenbrillen, Motorradbrillen, Visieren von Schutzhelmen, und dergleichen eingesetzt werden. Insbesondere können die erfindungsgemäßen Spirofluorenopyrane auch für Sonnenschutzzwecke in Fahrzeugen, Wohnungen in Form von Fenstern, Schutzblenden, Abdeckungen, Dächern oder dergleichen Verwendung finden.

Die erfindungsgemäßen Verbindungen zeichnen sich durch eine Vielzahl von Vorteilen aus: Es handelt sich um eine völlig neuartige Klasse photochromer Verbindungen, welche die Nachteile der im Stand der Technik bekannten Verbindungen nicht besitzen. In überraschender Weise konnten durch die erfindungsgemäßen Spiro-Verbindungen, d.h. durch Verknüpfung der üblicherweise am zentralen Kohlenstoffatom der Fluorenstruktur befindlichen freien Substituenten zu einem geschlossenen Ringsystem, wobei mindestens ein aromatisches und/oder heteroaromatisches System als zusätzlicher Bestandteil des neuen Rings vorhanden ist, die vorgenannten Probleme der Verbindungen aus dem Stand der Technik überwunden werden. Die im Stand der Technik beschriebenen nächstkommenden Verbindungen stellen die in der PCT/DE 98/02820 offenbarten indenoannellierten Naphthopyrane dar. Aus diesen Naphthopyranen können in Kenntnis der vorliegenden Erfindung Verbindungen in Form sogenannter "Carbinole" abgeleitet werden. Sowohl gegenüber den nächstkommenden bekannten Verbindungen als auch den abgeleiteten Verbindungen wurden bei den erfindungsgemäßen Spiro-Verbindungen eine wunschgemäße Beeinflussung der Aufhellung und wesentliche Verbesserungen in der Lebensdauer erzielt. Eine Vergilbung, wie sie bei den Carbinolen beobachtet wird, tritt bei den erfindungsgemäßen Spiro-Verbindungen nicht mehr auf. Völlig unerwartet ist dabei, daß sich trotz der elektronischen Entkoppelung der neuen erfindungsgemäßen Strukturteile vom Farbzentrum ein anderer, viel klarerer Farbeindruck in der angeregten Form einstellt.

Alle diese Spiro-Verbindungen der Erfindung zeichnen sich demzufolge gegenüber dem nächstkommenden Stand der Technik auch durch ein besseres Alterungsverhalten aus, sowohl bezüglich Leistungs- wie auch bezüglich Farberhalt. Da diese nächstkommenden Verbindungen der Klasse der indenoannellierten Naphthopyrane zuzurechnen sind, wurden diese im Stand der Technik besonders gewürdigt.

Die Eigenschaften der erfindungsgemäßen Spiro-Verbindungen sollen anhand einiger Verbindungen erläutert werden, wobei die Erfindung selbstverständlich nicht auf diese Ausführungsformen beschränkt wird.

<u>Aufhellungsgeschwindigkeit und längstwelliges Absorptionsmaximum $\lambda_{max}$</u>

Es wurde der Einfluß der Substituenten $R'_1$ - $R'_3$ auf die Aufhellungsgeschwindigkeit binnen 10 min ($\Delta_{10\ min} = [(\tau_{10\ min} - \tau_S) / (\tau_0 - \tau_S)]$) und das längstwellige Absorptionsmaximum $\lambda_{max}$ der erfindungsgemäßen Spiro-Verbindungen (X = Einfachbindung) untersucht. Die chemischen Strukturformeln der eingesetzten Spiro- und Carbinol-Verbindungen sind nachfolgend abgebildet, wobei $R_1'$ entweder Wasserstoff oder aus der Gruppe A ausgewählt ist, $R_2'$ und $R_3'$ unabhängig voneinander Wasserstoff oder aus der Gruppe F ausgewählt sind und $R_4'$ derart ausgewählt ist, daß sich durch Cyclisieren die entsprechende Spiro-Verbindung ergibt.

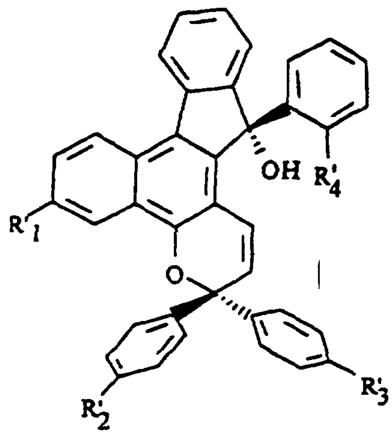

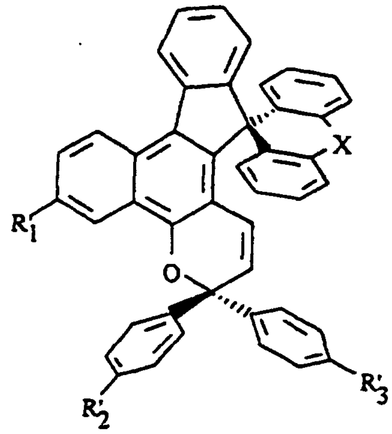

**Carbinole**
**(Stand der Technik)**

**Spiros**
**(erfindungsgemäße Spiro-Verbindungen)**

Die Ergebnisse der Messungen sind in Tabelle 1 zusammengestellt.

Tabelle 1

| | R'$_1$ | R'$_2$ | R'$_3$ | Beispiel | $\Delta_{10\ min}$ | $\lambda_{max}$ |
|---|---|---|---|---|---|---|
| A) Substituent R'$_1$ | | | | | | |
| | H | OMe | OMe | Spiro 10 | 54% | 550 nm |
| | OMe | OMe | OMe | Spiro 4 | 49% | 575 nm |
| | H | Morph | H | Spiro 11 | 44% | 575 nm |
| | OMe | Morph | H | Spiro 7 | 43% | 595 nm |
| B ) Substituent R'$_2$ | | | | | | |
| | OMe | H | H | Spiro 1 | 29% | 555 nm |
| | OMe | OMe | H | Spiro 2 | 37% | 565 nm |
| | OMe | Morph | H | Spiro 7 | 43% | 595 nm |
| | OMe | $NPh_2$ | H | Spiro 9 | 33% | 600 nm |
| | OMe | $NMe_2$ | H | Spiro 8 | 51% | 615 nm |
| C ) Substituent R'$_3$ | | | | | | |
| | OMe | OMe | H | Spiro 2 | 37% | 565 nm |
| | OMe | OMe | OMe | Spiro 4 | 49% | 575 nm |

Die erfindungsgemäßen Verbindungen sind in Tabelle 1 der Übersichtlichkeit halber durch "Spiro" abgekürzt, wobei sich die anschließende Zahl unmittelbar auf das entsprechende Beispiel, in dem die Herstellung dieser Verbindung beschrieben wird, bezieht.

Aus Tabelle 1 ist ersichtlich, daß ein elektronenspendender Substituent an R'$_1$ ($OCH_3$ statt H) $\lambda_{max}$ bathochrom um 20 - 25 nm verschiebt, wobei die Aufhellungsgeschwindigkeit in etwa gleich bleibt. Die gleiche Substitution an R'$_2$ führt zu einer bathochromen Verschiebung um nur 10 nm, dabei nimmt die Aufhellungsgeschwindigkeit um nahezu 30% zu. Mit stärker elektronenspendenden Substituenten läßt sich $\lambda_{max}$ bis zu 50 nm bathochrom verschieben. Eine weitere $OCH_3$-Gruppe statt H an R'$_3$ ergibt wieder eine weitere bathochrome Verschiebung um 10 nm und eine Erhöhung der Aufhellungsgeschwindigkeit um über 30%. Wie gezeigt wird, läßt sich durch die erfindungsgemäße Lehre sowohl das längstwellige Absorptionsmaximum wie auch die Aufhellgeschwindigkeit durch geeignete Wahl der Substituenten $R'_1$ - $R'_3$ in einem sehr weiten Bereich auf den jeweiligen Anwendungszweck maßgeschneidert abstimmen.

Im Folgenden wurde der Einfluß der Spiroverknüpfung auf die Aufhellungsgeschwindigkeit binnen 10 min ($\Delta_{10\ min} = [(\tau_{10\ min} - \tau_s) / (\tau_0 - \tau_s)]$) und das längstwellige Absorptionsmaximum $\lambda_{max}$ untersucht. Carbinol-Verbindungen wurden der entsprechenden erfindungsgemäßen Spiro-Verbindung gegenübergestellt und die erhaltenen Daten verglichen.

Die erhaltenen Ergebnisse sind in Tabelle 2 wiedergegeben.

Tabelle 2 :

| $R'_1$ | $R'_2$ | $R'_3$ | $R'_4$ | X | Beispiel | $\Delta_{10\ min}$ | $\lambda_{max}$ |
|---|---|---|---|---|---|---|---|
| | | | | | | | |
| OMe | H | H | Ph | | Carbinol 1 | 22 % | 560 nm |
| OMe | H | H | | -* | Spiro 1 | 29% | 555 nm |
| | | | | | | | |
| OMe | OMe | H | Ph | | Carbinol 2 | 26% | 575 nm |
| OMe | OMe | H | | - | Spiro 2 | 37% | 565 nm |
| | | | | | | | |
| OMe | OMe | H | OPh | | Carbinol 3 | 25% | 575 nm |
| OMe | OMe | H | | O | Spiro 3 | 26 % | 565 nm |
| | | | | | | | |
| OMe | OMe | OMe | Ph | | Carbinol 4 | 36% | 585 nm |
| OMe | OMe | OMe | | - | Spiro 4 | 49% | 575 nm |
| | | | | | | | |
| OMe | OMe | OMe | OPh | | Carbinol 5 | 35% | 585 nm |
| OMe | OMe | OMe | | O | Spiro 5 | 35% | 575 nm |
| | | | | | | | |
| OMe | OMe | OMe | $CH_2Ph$ | | Carbinol 6 | 44% | 580 nm |
| OMe | OMe | OMe | | $CH_2$ | Spiro 6 | 29% | 575 nm |
| | | | | | | | |
| OMe | Morph | H | Ph | | Carbinol 7 | 25% | 600 nm |
| OMe | Morph | H | | - | Spiro 7 | 43% | 595 nm |

* "-" bedeutet, daß X eine Einfachbindung darstellt.

Die an den Stand der Technik angelehnten Verbindungen sind in Tabelle 2 der Übersichtlichkeit halber durch "Carbinol" abgekürzt, wobei sich die anschließende Zahl unmittelbar auf das entsprechende Beispiel, in dem die Herstellung dieser Verbindung beschrieben wird, bezieht.

Wie aus Tabelle 2 ersichtlich, ist der Einfluß der Spiroverknüpfung auf das längstwellige Absorptionsmaxima sehr gering. Gegenüber den baugleichen Carbinol-Ausgangsverbindungen ergibt sich lediglich eine hypsochrome Verschiebung um 5-10 nm. Die Aufhellgeschwindigkeit kann dagegen wunschgemäß je nach Art der Spiroverknüpfung beeinflußt werden: Eine direkte Verknüpfung beschleunigt, eine Sauerstoffbrücke läßt unverändert und eine Methylenbrücke verlangsamt die Aufhellgeschwindigkeit.

Dies ist in Figur 1 für die Spiro-Verbindungen Spiro 4-6 graphisch anschaulich gezeigt. Die Meßung erfolgte bei 23°C, 15 min. Belichtung, bei 50 klux und 10 min. Dunkelaufhellung. Hierbei wurden die unterschiedlichen Transmissionswerte nach 15 min Belichtung zum einfacheren Vergleich in äquidistante Abstände gebracht.

Es ist nunmehr mit der erfindungsgemäßen Lehre möglich, nachdem man mit Hilfe von $R'_1$ - $R'_3$ die sonstigen Eigenschaften der photochromen Verbindung (u.a. Absorptionsverhalten, Migrationsverhalten, Affinität zur Polymermatrix) auf den jeweiligen Anwendungszweck optimal angepaßt hat, mittels $R'_4$ die Kinetik, d.h. die Aufhellgeschwindigkeit und damit die Temperaturabhängigkeit der photochromen Reaktion, wunschgemäß anzupassen. Bei der Auswahl der Farbe ist nur die leichte hypsochrome Verschiebung durch die Spiroverknüpfung bei der Auswahl von $R'_1$ - $R'_3$ von vorneherein mit zu berücksichtigen.

Dieser völlig überraschende Effekt der unterschiedlichen Spiroverknüpfungsbrücken ist für die erfindungsgemäßen Spiro-Verbindungen ebensowenig vorhersehbar wie das stark verbesserte Verhalten im Lebensdauertest.

Gelbindexzunahme Δyi sowie Leistungserhalt LE im Lebensdauertest

Ferner wurden die Gelbindexzunahme Δyi sowie der Leistungserhalt LE im Lebensdauertest (50h Xenonlampe) bestimmt. Es gilt:

$$\Delta yi = yi_{\text{nach 50th Xenontest}} - yi_{\text{vor Xenontest:}}\ LE = (\tau_{\text{S vor Xenontest}} / \tau_{\text{S nach 50h Xenontest}})$$

Die eingesetzten Verbindungen sind nachfolgend anhand ihrer chemischen Strukturformeln dargestellt.

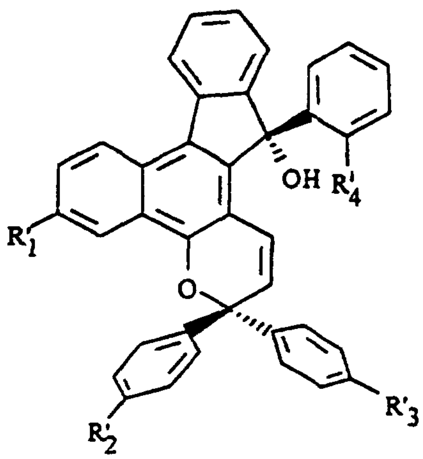

**Carbinole**

**(Stand der Technik)**

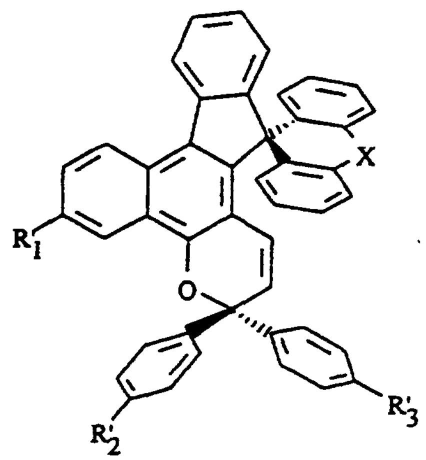

**Spiros**

**(erfindungsgemäße Verbindungen)**

Die erhaltenen Ergebnisse gehen aus Tabelle 3 hervor.

Tabelle 3:

| $R'_1$ | $R'_2$ | $R'_3$ | $R'_4$ | X | Beispiel | Δyi | LE |
|---|---|---|---|---|---|---|---|
| | | | | | | | |
| OMe | H | H | Ph | | Carbinol 1 | 7,22 | 89% |
| OMe | H | H | | -* | Spiro 1 | -0,10 | 90% |
| | | | | | | | |
| OMe | OMe | H | Ph | | Carbinol 2 | 15,82 | 85% |
| OMe | OMe | H | | - | Spiro 2 | 1,68 | 89% |
| | | | | | | | |
| OMe | OMe | H | OPh | | Carbinol 3 | 3,46 | 92% |

* "-" bedeutet, daß X eine Einfachbindung darstellt.

Tabelle 3: (fortgesetzt)

| R'$_1$ | R'$_2$ | R'$_3$ | R'$_4$ | X | Beispiel | Δyi | LE |
|---|---|---|---|---|---|---|---|
| OMe | OMe | H | | O | Spiro 3 | -0,57 | 90% |
| | | | | | | | |
| OMe | OMe | OMe | Ph | | Carbinol4 | 29,86 | 77% |
| OMe | OMe | OMe | | - | Spiro 4 | -0,97 | 98% |
| | | | | | | | |
| OMe | OMe | OMe | OPh | | Carbinol 5 | 6,75 | 86% |
| OMe | OMe | OMe | | O | Spiro 5 | -2,29 | 85% |
| | | | | | | | |
| OMe | OMe | OMe | $CH_2Ph$ | | Carbinol 6 | 32,34 | 81% |
| OMe | OMe | OMe | | $CH_2$ | Spiro 6 | -1,92 | 98% |
| | | | | | | | |
| OMe | Morph | H | Ph | | Carbinol 7 | 19,69 | 80% |
| OMe | Morph | H | | - | Spiro 7 | 0,25 | 89% |

Gegenüber den entsprechenden Carbinolverbindungen, die strukturell dem Stand der Technik nahekommen, wird allgemein bei den erfindungsgemäßen Spiro-Verbindungen ein besserer Erhalt der photochromen Leistung erzielt. Im Einzelfall, wie bei Beispiel 4 und 6, beträgt der Leistungsverlust nach 50 h Xenontest nur 2% für die Spiro-Verbindungen, aber 20% für die Carbinole. Im Langzeiteinsatz in ophthalmischen Gläsern kann der Brillenträger daher mit den erfindungsgemäßen Verbindungen mit einem Vielfachen der Tragedauer rechnen.

Noch dramatischer und ebenfalls völlig überraschend ist die Verringerung der Gelbindexveränderung im voll eingedunkelten Zustand nach 50 h Xenontest, in allen Fällen. Dies war nicht zu erwarten und ist auch nicht auf das Fehlen der Hydroxygruppe in der Spiro-Verbindung zurückzuführen, da einige Carbinole nur eine geringe (Nr. 3), andere dagegen eine extreme Gelbindexveränderung (Nr. 6) zeigen.

In den Figuren 2, 3 und 4 sind die a*/b*-Farbortkurven von drei Carbinol- bzw. Spiro-Verbindungen vor und nach 50 h Xenontest abgebildet. Die Messungen erfolgten bei 23 °C, 15 min. Belichtung, bei 50 Klux und 10 min. Dunkelaufhellung. Hier ist deutlich zu sehen, daß sich der Farbort der Carbinole im gesamten Verlauf eines Eindunkelungs- und Aufhellzyklus durch die Alterung dramatisch verschiebt, während die entsprechenden Spiroverbindungen über die Alterung farbtreu bleiben. Dies wird eindrucksvoll durch die Lage der Meßkurven der Carbinol- bzw. Spiro-Verbindung vor und nach dem Xenon-Alterungstest belegt.

Für den Brillenträger sind zwei Zustände von besonderer Bedeutung: der aufgehellte und der voll eingedunkelte Zustand. Im Gegensatz zu den Zwischenzuständen werden diese quasi-stationär erlebt. Alle drei beispielhaften Brückensysteme der erfindungsgemäßen Spiroverbrückung zeigen gegenüber den entsprechenden Carbinolen praktisch keine Alterungstendenz. Dies ist für den Einsatz in Brillen noch wichtiger als der reine Leistungserhalt, da der Brillenträger erfahrungsgemäß ein geringfügiges Nachlassen der Eindunkelungsleistung im längeren Gebrauch nicht bemerkt. Eine geringfügige Farbänderung wird dagegen schnell bemerkt und als Mangel reklamiert.

Nachfolgend wird die Herstellung der Spiro-Verbindungen der Erfindung anhand des allgemeinen Synthesewegs erläutert. Hierzu wird auf das allgemeine Syntheseschema, das in den Figuren 5 und 6 dargestellt ist, Bezug genommen:

Die als Ausgangsmaterial verwendeten substituierten oder unsubstituierten Benzo- bzw. Naphthophenone (a) sind entweder im Handel erhältlich oder können durch Friedel-Crafts-Acylierung gemäß Schritt (1) leicht und meist in guten Ausbeuten erhalten werden. Die Reste $R_3$ und $R_4$ entsprechen hierbei den oben bereits definierten Substituenten. $(R_1')_m$ ist ausgewählt aus der Gruppe A, wobei m = 0, 1 oder 2 ist.

In Schritt (2) wird über eine Stobbe-Kondensation mittels Bernsteinsäurediester der Halbester (b) erzeugt. Sofern die beiden über die Ketogruppe verbundenen Ringe nicht identisch sind, entsteht ein Isomerengemisch. Abweichend von der Darstellung in der WO 96/14596 wird in der vorliegenden Erfindung mit dem Isomerengemisch weitergearbeitet. Die Trennung der honigartigen, meist nicht kristallisierenden Isomeren ist sehr aufwendig, sie kann auf dem erfindungsgemäß gewählten Syntheseweg später sehr einfach durchgeführt werden. Anschließend wird das Halbestergemisch mit wäßrigem Alkali gemäß Schritt (3) zu dem isomeren Gemisch der Disäuren (c) verseift. Durch Erwärmen mit Acetylchlorid in Schritt (4) wird dann unter Wasserabspaltung das Isomerengemisch der cyclischen

Anhydride (d) hergestellt.

In Schritt (5) wird mit Aluminiumchlorid durch intramolekulare 5-Ring-Cyclisierung das Isomerengemisch (e) synthetisiert. Diese Verbindungen kristallisieren sehr gut und können in den meisten Fällen dank ihrer unterschiedlichen Löslichkeit in Benzol leicht in die beiden Isomeren getrennt werden. Aus dem gewünschten Isomer entsteht unter dem Einfluß von Acetanhydrid durch intramolekulare 6-Ring-Cyclisierung gemäß Schritt (6) der Ester (f). Dieser wird anschließend in Schritt (7) alkalisch zum Hydroxy-fluorenon-Derivat (g) verseift.

Die Umsetzung mit einem 2-Propin-1-ol-derivat (Schritt (8)), in dem B und B' wie oben definert sind, führt zu den schwach photochromen, stark dunkelrot gefärbten indenoinnellierten Naphthopyranen (h). Diese Umsetzung wird hier nicht näher beschrieben, da sie sich in analoger Weise wie bei den 1- oder 2-Naphtholen durchführen läßt. Beispiele hierzu finden sich u.a. in der US 5.066.818, der US 5.238.981 und der EP 0 246 114. Mit entsprechenden Grignard-Verbindungen entstehen in Schritt (9) die in der vorliegenden Erfindung als sogenannte Carbinole bezeichneten Verbindungen (i), wobei $R'_4$ derart ausgewählt wird, daß die entsprechenden Spiro-Verbindungen mit dem oben definierten Ring G gebildet werden können. Die freie Hydroxylgruppe in (i) wird anschließend gemäß Schritt (10) mit Essigsäure/ Salzsäure zu den entsprechenden Spiro-Verbindungen (k) cyclisiert.

Der vorgenannte Syntheseweg weist nun gegenüber dem in der WO 96/14596 bzw. der US 5.645.767 empfohlenen Weg verschiedene Vorteile auf:

Zum einen sind verschiedene Verbindungen auf dem in der WO 96/14596 bzw. der US 5.645.767 beschriebenen Weg nicht darstellbar. Der oben beschriebene Weg ist wesentlich schonender, so entfällt beispielsweise das einstündige Erhitzen mit konzentrierter Phosphorsäure auf über 190°C. Dadurch sind als Substituenten auch empfindlichere Gruppierungen einsetzbar. Die Schließung des 5-Ringes vor dem 6-Ring im obigen Verfahren führt ebenfalls häufig zu Produkten, die auf dem im Stand der Technik genannten Weg - Schließung des 6-Rings vor dem 5-Ring - nicht oder nur schwer darstellbar sind. Zum anderen gibt es selbst bei gleichem Zielprodukt, z.B. bei symmetrischen Benzophenonen als Ausgangsmaterial, nach dem obigen beschriebenen Verfahren weniger Nebenprodukte, bei einfacherer Reinigung und höheren Ausbeuten des photochromen Farbstoffs. Das genaue synthetische Vorgehen ist bei der Darstellung der nachfolgenden Beispiele detailliert beschrieben, wobei diese Beispiele selbstverständlich den Schutzbereich der vorliegenden Erfindung nicht beschränken, sondern nur zur Veranschaulichung dienen sollen.

<u>BEISPIEL 1</u>

Die Schritte i) bis vi) dieses Beispiels wurden auf der Grundlage des in J.Chem.Soc. 1958, S. 986ff von F.G. Baddar, L.S.El-Assal und V.B.Baghos publizierten Artikels mit einigen Modifikationen durchgeführt.

i) tert. Butanol wird im warmen Wasserbad vorab geschmolzen. Kalium-tert.-butylat (30 g) wird in einem 1l-Dreihalskolben in 600 ml tert. Butanol suspendiert und 4-Methoxybenzophenon (50 g) sowie Bernsteinsäuredimethylester (45 g) zugefügt. Die Mischung wird gut gerührt und 1 h unter Rückfluß erhitzt. Danach gibt man nochmals Kalium-tert.-butylat (30 g) sowie Bernsteinsäuredimethylester (45 g) zu und läßt 2 h unter Rückfluß erhitzen. Nach dem Abkühlen wird mit insgesamt 2 l Wasser hydrolysiert. Unter Rühren wird mit konz. Salzsäure angesäuert. Anschließend wird zweimal mit je 400 ml Ether extrahiert, und die vereinigten Ether-Phasen werden einmal mit 400 ml Wasser gewaschen. Dann wird zweimal mit 500 ml gesättigter Natriumhydrogencarbonat-Lösung extrahiert. Dabei geht das Produkt als Carboxylat in die wäßrige Phase, die einmal mit 200 ml Ether gewaschen und anschließend in einem großen Becherglas unter Rühren mit konz. Salzsäure angesäuert wird. Anschließend wird zweimal mit je 400 ml Ether extrahiert und die organische Phase einmal mit Wasser gewaschen. Nach Trocknen über Natriumsulfat wird der Ether gründlich abrotiert. Das orangegelbe, honigartige Reaktionsprodukt (75 g) wurde mittels NMR-Spektrum als 3-Methoxycarbonyl-4-(4-methoxyphenyl)-4-phenyl-3-butensäure identifiziert.

ii) In einem 1l-Kolben wird das obige Reaktionsprodukt in einer Lösung von Kaliumhydroxid (40 g) in etwa 600 ml Wasser aufgelöst. Die gebildete braune Reaktionslösung wird 3 h unter Rückfluß erhitzt. Nach dem Abkühlen wird unter Rühren und guter Kühlung im Eisbad mit konz. Salzsäure angesäuert. Anschließend wird zweimal mit je 400 ml Essigester extrahiert. Die vereinigten Essigester-Phasen werden einmal mit Wasser gewaschen und nach dem Trocknen über Natriumsulfat gründlich abrotiert. Das honigartige Reaktionsprodukt (70 g) wurde mittels NMR-Spektrum als 3-Carboxyl-4-(4-methoxyphenyl)-4-phenyl-3-butensäure identifiziert.

iii) Das obige Reaktionsprodukt wird mit Acetylchlorid (35 g) in 300 ml Essigsäure gelöst. Unter Rühren wird nun 2 h unter Rückfluß erhitzt. Nach Abdestillieren des Solvens wird der Rückstand noch warm in etwa 600 ml Essigester gelöst und zweimal mit Wasser extrahiert. Anschließend wird zweimal mit je 300 ml 5 % iger Natriumcarbonat-Lösung extrahiert. Die organische Phase wird nochmals mit Wasser gewaschen, über Natriumsulfat getrocknet und gründlich abrotiert. Das Reaktionsprodukt (60 g) fällt als dunkles, viskoses Öl an und wurde mittels NMR-Spektrum als (4-Methoxyphenyl-phenyl-methylen)bernsteinsäureanhydrid identifiziert.

iv) Das obige Reaktionsprodukt wird in etwa 600 ml Dichlormethan bei Raumtemperatur gelöst und anschließend im Eisbad gut gekühlt. Anschließend wird unter gutem Rühren und Kühlen portionsweise Aluminiumchlorid (35 g) zugegeben und man läßt die Mischung über Nacht auftauen. Danach wird die Reaktionsmischung zur Hydrolyse in 1 l Eis/Wasser gegossen. Nach Abtrennen der organischen Phase wird diese zweimal mit je 500 ml Wasser gewaschen und anschließend zweimal mit 600 ml 5%iger Natronlauge extrahiert. Nach Waschen der vereinigten wäßrig-alkalischen Phasen mit 250 ml Ether wird mit konz. Salzsäure unter Rühren angesäuert. Es wird zweimal mit je 400 ml Essigester extrahiert und die organische Phase mit Wasser gewaschen, über Natriumsulfat getrocknet und gründlich abrotiert. Es hinterbleibt ein brauner fester Rückstand (45 g), der laut NMR-Spektrum aus dem Isomerengemisch 3-(4-Methoxy-phenyl)-indenon-2-essigsäure und 6-Methoxy-3-phenyl-indenon-2-essigsäure besteht. Die Isomerentrennung gelingt durch heißes Digerieren in Benzol. Nach Abkühlen und Absaugen der Suspension erhält man als Rückstand reine 3-(4-Methoxyphenyl)-indenon-2-essigsäure als orangegelbe Kristalle (25 g).

v) Das obige Reaktionsprodukt wird in etwa 300 ml Acetanhydrid suspendiert. Nach Zugabe von Natriumacetat (20 g) wird die Mischung 3 h unter Rühren zum Rückfluß erhitzt. Das Produkt fällt beim Abkühlen dick aus. Nach Abkühlen auf Raumtemperatur (und noch kurz im Kühlschrank) wird der gebildete weiche, kristalline, orangebräunliche Niederschlag abgesaugt und mit wenig Acetanhydrid gewaschen, bis das Filtrat nicht mehr dunkelbraun abläuft. Anschließend wird gründlich mit Wasser gewaschen und bei 60° C getrocknet. Das Produkt (leuchtend orangefarbener Feststoff; 25 g) wurde mittels NMR-Spektrum als 5-Acetoxy-3-methoxy-7H-benzo[c]fluoren-7-on identifiziert.

vi) Das obige Reaktionsprodukt wird in 400 ml Ethanol suspendiert und mit Kaliumhydroxid (25 g) versetzt. Die Reaktionsmischung wird 1,5 h unter Rühren zum Rückfluß erhitzt, wobei sich allmählich eine prächtige tiefblaue Farbe beobachten läßt. Nach Abkühlen wird etwa die Hälfte des Ethanols abrotiert und der verbleibende Rückstand zusammen mit 1 l Wasser auf der Heizplatte erhitzt, bis sich eine tiefblaue Lösung gebildet hat. Dann wird die Lösung von der Heizplatte genommen und noch heiß unter Rühren mit konz. Salzsäure angesäuert. Es entsteht eine dunkelviolette Suspension, die unter Rühren auf Raumtemperatur abgekühlt wird. Die Suspension wird abgesaugt und sorgfältig mit Wasser gewaschen. Der abgenutschte Stoff ist noch sehr schlammig und wird so gut wie möglich an der Membranpumpe trockengesaugt. Nach dem Trocknen bei 60°C wurde das rotviolette Produkt (20 g) mittels NMR-Spektrum als 5-Hydroxy-3-methoxy-7H-benzo[c]fluoren-7-on identifziert.

vü) 3 g des obigen Reaktionsproduktes werden zusammen mit 1,1-Diphenyl-1-propinol (4 g; hergestellt aus Benzophenon und Natriumacetylid in DMSO) in etwa 300 ml Toluol suspendiert. Nach Zusatz einer Spatelspitze 4-Toluolsulfonsäure wird die Reaktionsmischung 1.5 h unter Rückfluß erhitzt. Im Laufe der Reaktion geht das zunächst schlechtlösliche Naphthol-Edukt immer mehr in Lösung; es entsteht eine rote Reaktionslösung. Nach kurzem Abkühlen wird das Toluol im Vakuum abrotiert und der Rückstand in 40 ml Dichlormethan gelöst und einer Säulenchromatographie mit Aluminiumoxid (Wassergehalt 3%) als stationärer und Dichlormethan/Hexan-Mischung (2:1) als mobiler Phase unterzogen. Zur endgültigen Reinigung wird das Produkt in etwa 100 ml Methanol digeriert und leicht erwärmt. Nach Abkühlen wird die entstandene Suspension abgesaugt, mit Methanol gewaschen und getrocknet. Das dunkelrote Produkt (3 g) wurde mittels NMR-Spektrum als 3.3-Diphenyl-6-methoxy-13-oxo-indeno [2,1-f]naphtho[1,2-b]pyran identifiziert.

viii) 2 g des obigen Reaktionsproduktes wird in 50 ml absolutem THF unter Rühren gelöst. Zu dieser Lösung werden 2 Äquivalente 2-Biphenylyl-magnesiumbromid (hergestellt aus 2-Brombiphenyl und Magnesiumspänen in THF-Lösung) zugetropft und das ganze bei Rzumtemperatur 1 h gerührt. Anschließend gießt man in Wasser, säuert mit konz. Salzsäure solange an, bis die Phasen klar sind und trennt die organische Phase ab. Nach Extraktion mit Wasser, Trocknen über Natriumsulfat und Abrotieren des Solvens hinterbleibt ein dunkelbraunes Öl, das durch Zugabe von Methanol kristallisiert. Kristallisiert das Produkt nicht, wird solange im Eisbad gerührt, bis Kristalle ausfallen. Man rührt die Suspension noch einige Minuten bei Raumtemperatur, saugt dann den Niederschlag ab und wäscht mit Methanol nach. Der entstandene beigefarbene Feststoff (1 g) wurde mittels NMR-Spektrum als 13-(2-Biphenylyl)-3,3-diphenyl-13-hydroxy-6-methoxy-indeno[2,1-f]naphtho[1,2-b]pyran identifiziert (Carbinol 1).

ix) 1g des obigen Reaktionsproduktes werden gemäß R.G.Clarkson, M.Gomberg, J.Am.Chem.Soc. 1930, S.2881ff in 30 ml Eisessig in der Hitze cyclisiert. Nach Zugabe eines Tropfens Salzsäure wird noch 5 min zum Sieden erhitzt und noch heiß solange Wasser zugegeben, bis die Reaktionslösung trüb wird. Nach dem Abkühlen wird der ausgefallene Niederschlag abgesaugt, mit Wasser gewachen und sorgfältig getrocknet. Zur endgültigen Reinigung wird der Feststoff in 40 ml Dichlormethan gelöst und einer Säulenchromatographie mit Aluminiumoxid (Wassergehalt 3%) als stationärer und Dichlormethan/Hexan-Mischung (2:1) als mobiler Phase unterzogen. Nach Digerieren mit Methanol wird das reine leicht beigefarbene Produkt (0.5 g) erhalten, das mittels NMR-Spektrum als Spiro-9-fluoren-13'-[3,3-diphenyl-6-methoxy-indeno[2,1-f]naphtho[1,2-b]pyran] identifiziert wurde (Spiro 1).

BEISPIEL 2

Die Durchführung erfolgt analog Beispiel 1, nur im Schritt vii) erfolgt die Umsetzung mit 1-(4-Methoxyphenyl)-1-phenyl-1-propinol (hergestellt aus 4-Methoxybenzophenon und Natriumacetylid in DMSO) statt 1,1-Diphenyl-1-propinol. Es entsteht 6-Methoxy-3-(4-methoxyphenyl)-3-phenyl-13-oxo-indeno[2,1-f]naphtho[1,2-b]pyran. Dieses wird analog zu Beispiel 1 Schritt viii) mit 2-Biphenylyl-magnesiumbromid laut NMR-Spektrum zu 13-(2-Biphenylyl)-13-hydroxy-6-methoxy-3-(4-methoxyphenyl)-3-phenyl-indeno[2,1-f]naphtho[1,2-b]pyran umgesetzt (Carbinol 2). Dieses wird analog Beispiel 1 Schritt ix) cyclisiert zu Spiro-9-fluoren-13'-[6-methoxy-3-(4-methoxyphenyl)-3-phenyl-indeno [2,1-f]naphtho[1,2-b]pyran] (Spiro 2; 0.7 g), das mittels NMR-Spektrum identifiziert wurde.

BEISPIEL 3

Die Durchführung erfolgt analog Beispiel 2, nur im Schritt viii) erfolgt die Umsetzung mit 2-Phenoxyphenylmagnesiumbromid (hergestellt aus 2-Bromdiphenylether und Magnesium in THF-Lösung) statt 2-Biphenylylmagnesiumbromid . Es entsteht laut NMR-Spektrum 13-Hydroxy-6-methoxy-3-(4-methoxyphenyl)-13-(2-phenoxyphenyl)-3-phenyl-indeno[2,1-f]-naphtho[1,2-b]pyran (Carbinol 3). Dieses wird analog Beispiel 1 Schritt ix) cyclisiert zu Spiro-9-xanthen-13'-[6-methoxy-3-(4-methoxyphenyl)-3-phenyl-indeno[2,1-f]naphtho[1,2-b]pyran] (Spiro 3; 0.4 g), das mittels NMR-Spektrum identifiziert wurde.

BEISPIEL 4

Die Durchführung erfolgt analog Beispiel 1, nur im Schritt vii) erfolgt die Umsetzung mit 1,1-Bis(4-methoxyphenyl)-1-propinol (hergestellt aus 4,4'-Dimethoxybenzophenon und Natriumacetylid in DMSO) statt 1,1-Diphenyl-1-propinol. Es entsteht 3,3-Bis(4-methoxyphenyl)-6-methoxy-13-oxo-indeno[2,1-f]naphtho[1,2-b]pyran. Dieses wird analog zu Beispiel 1 Schritt viii) mit 2-Biphenylyl-magnesiumbromid laut NMR-Spektrum zu 13-(2-Biphenylyl)-3,3-bis (4-methoxyphenyl)-13-hydroxy-6-methoxy-indeno[2,1-f]naphtho[1,2-b]pyran umgesetzt (Carbinol 4). Dieses wird analog Beispiel 1 Schritt ix) cyclisiert zu Spiro-9-fluoren-13'-[3,3-bis(4-methoxyphenyl)-6-methoxy-indeno[2,1-f]naphtho [1,2-b]pyran] (Spiro 4; 0.6 g), das mittels NMR-Spektrum identifiziert wurde.

BEISPIEL 5

Die Durchführung erfolgt analog Beispiel 4, nur im Schritt viii) erfolgt die Umsetzung mit 2-Phenoxyphenylmagnesiumbromid statt 2-Biphenylyl-magnesiumbromid. Es entsteht laut NMR-Spektrum 3,3-Bis(4-methoxyphenyl)-13-hydroxy-6-methoxy-13-(2-phenoxy-phenyl)-indeno[2,1-f]naphtho[1,2-b]pyran (Carbinol 5). Dieses wird analog Beispiel 1 Schritt ix) cyclisiert zu Spiro-9-xanthen-13'-[3,3-bis(4-methoxyphenyl)-6-methoxy-indeno[2,1-f]naphtho[1,2-b] pyran] (Spiro 5; 0.9 g), das mittels NMR-Spektrum identifiziert wurde.

BEISPIEL 6

Die Durchführung erfolgt analog Beispiel 4, nur im Schritt viii) erfolgt die Umsetzung mit 2-Benzylphenylmagnesiumbromid (hergestellt aus 2-Bromdiphenylmethan und Magnesium in THF-Lösung) statt 2-Biphenylyl-magnesiumbromid. Es entsteht laut NMR-Spektrum 13-(2-Benzylphenyl) 3,3-bis(4-methoxyphenyl)-13-hydroxy-6-methoxy-indeno[2,1-f]naphtho-[1,2-b]pyran (Carbinol 6). Dieses wird analog Beispiel 1 Schritt ix) cyclisiert zu Spiro-9-(9,10-dihydroanthracen)-13'-[3,3-bis(4-metboxyphenyl)-6-methoxy-indeno[2,1-f]naphtho-[1,2-b]pyran] (Spiro 6; 0.5 g), das mittels NMR-Spektrum identifiziert wurde.

BEISPIEL 7

Die Durchführung erfolgt analog Beispiel 1, nur im Schritt vii) erfolgt die Umsetzung mit 1-[4-(N-Morpholinyl) phenyl]-1-phenyl-1-propinol (hergestellt aus 4-(N-Morpholinyl)-benzophenon (H.Kotsuki, Synthesis 1990, S.1145) und Natriumacetylid in DMSO] statt 1,1-Diphenyl-1-propinol. Es entsteht 6-Methoxy-3-[4-(N-morpholinyl)phenyl]-3-phenyl-13-oxoindeno[2,1-f]naphtho[1,2-b]pyran. Dieses wird analog zu Beispiel 1 Schritt viii) mit 2-Biphenylyl-magnesiumbromid laut NMR-Spektrum zu 13-(2-Biphenylyl)-13-hydroxy-6-methoxy-3-[4-(N-morpholinyl)phenyl]-3-phenyl-indeno [2,1-f]naphtho[1,2-b]pyran umgesetzt (Carbinol 7). Dieses wird analog Beispiel 1 Schritt ix) cyclisiert zu Spiro-9-fluoren-13'-{6-methoxy-3-[4-(N-morpholinyl)phenyl]-3-phenyl-indeno[2,1-f]naphtho[1,2-b]pyran} (Spiro 7; 0.4 g), das mittels NMR-Spektrum identifiziert wurde.

BEISPIEL 8

Die Durchführung erfolgt analog Beispiel 1, nur im Schritt vii) erfolgt die Umsetzung mit 1-(4-Dimethylaminophenyl)-1-phenyl-1-propinol (hergestellt aus 4-Dimethylamino-benzophenon und Natriumacetylid in DMSO) statt 1,1-Diphenyl-1-propinol. Es entsteht 3-(4-Dimethylaminophenyl)-6-methoxy-3-phenyl-13-oxo-indeno[2,1-f]naphtho [1,2-b]pyran. Dieses wird analog zu Beispiel 1 Schritt viii) mit 2-Biphenylyl-magnesiumbromid laut NMR-Spektrum zu 13-(2-Biphenylyl)-3-(4-dimethylaminophenyl)-13-hydroxy-6-methoxy-3-phenyl-indeno[2,1-f]naphtho[1,2-b]pyran umgesetzt. Dieses wird analog Beispiel 1 Schritt ix) cyclisiert zu Spiro-9-fluoren-13'-[3-(4-dimethylaminophenyl)-6-methoxy-3-phenyl-indeno[2.1-f]naphtho[1,2-b]pyran] (Spiro 8; 0.6 g), das mittels NMR-Spektrum identifiziert wurde.

BEISPIEL 9

Die Durchführung erfolgt analog Beispiel 1, nur im Schritt vii) erfolgt die Umsetzung mit 1-(4-Diphenylaminophenyl)-1-phenyl-1-propinol [hergestellt aus 4-Diphenylamino-benzophenon (B.Staskun, J.Org.Chem. 1968, S.3031) und Natriumacetylid in DMSO] statt 1,1-Diphenyl-1-propinol. Es entsteht 3-(4-Diphenylaminophenyl)-6-methoxy-3-phenyl-13-oxoindeno[2,1-f]naphtho[1,2-b]pyran. Dieses wird analog zu Beispiel 1 Schritt viii) mit 2-Biphenylyl-magnesiumbromid laut NMR-Spektrum zu 13-(2-Biphenylyl)-3-(4-diphenylaminophenyl)-13-hydroxy-6-methoxy-3-phenyl-indeno[2,1-f]naphtho[1,2-b]pyran umgesetzt. Dieses wird analog Beispiel 1 Schritt ix) cyclisiert zu Spiro-9-fluoren-13'-[3-(4-diphenylaminophenyl)-6-methoxy-3-phenyl-indeno[2,1-f]naphtho[1,2-b]pyran] (Spiro 9; 0.5 g), das mittels NMR-Spektrum identifiziert wurde.

BEISPIEL 10

Die Durchführung erfolgt analog Beispiel 4, nur im Schritt i) erfolgt die Umsetzung mit Benzophenon statt 4-Methoxybenzophenon. Es entsteht nach Schritt vii) 3,3-Bis(4-methoxyphenyl)-13-oxo-indeno[2,1-f]naphtho[1,2-b] pyran. Dieses wird analog zu Beispiel 1 Schritt viii) mit 2-Biphenylyl-magnesiumbromid laut NMR-Spektrum zu 3,3-Bis (4-methoxyphenyl)-13-(2-biphenylyl)-13-hydroxy-indeno[2,1-f]naphtho[1,2-b]pyran umgesetzt. Dieses wird analog Beispiel 1 Schritt ix) cyclisiert zu Spiro-9-fluoren-13'-[3,3-bis(4-methoxyphenyl)-indeno[2,1-f]naphtho[1,2-b]pyran] (Spiro 10; 0.7 g), das mittels NMR-Spektrum identifiziert wurde.

BEISPIEL 11

Die Durchführung erfolgt analog Beispiel 10, nur im Schritt vii) erfolgt die Umsetzung mit 1-[4-(N-Morpholinyl) phenyl]-1-phenyl-1-propinol statt 1,1-Bis(4-methoxyphenyl)-1-propinol. Es entsteht 3-[4-(N-Morpholinyl)phenyl]-3-phenyl-13-oxo-indeno[2,1-f]naphtho[1,2-b]pyran. Dieses wird analog zu Beispiel 1 Schritt viii) mit 2-Biphenylyl-magnesiumbromid laut NMR-Spektrum zu 13-(2-Biphenylyl)-13-hydroxy-3-[4-(N-morpholinyl)phenyl]-3-phenyl-indeno [2,1-f]naphtho[1,2-b]pyran umgesetzt. Dieses wird analog Beispiel 1 Schritt ix) cyclisiert zu Spiro-9-fluoren-13'-{3-[4-(N-morpholinyl)phenyl]-3-phenyl-indeno[2,1-f]naphtho[1,2-b]pyran} (Spiro 11; 0.6 g), das mittels NMR-Spektrum identifiziert wurde.

BEISPIEL 12

Die Durchführung erfolgt analog Beispiel 4 unter Weglassen der Schritte i) bis vi). Als Ausgangsmaterial für Schritt vii) wird 3-Brom-2-hydroxyfluorenon (T.C.Thomas, J.Indian Chem.Soc. 1974, S.814) statt 5-Hydroxy-3-methoxy-7H-benzo[c]fluoren-7-on verwendet. Es entsteht 3,3-Bis(4-methoxyphenyl)-5-brom-11-oxo-fluoreno[2,1-b]pyran. Dieses wird analog zu Beispiel 1 Schritt viii) mit 2-Biphenylyl-magnesiumbromid laut NMR-Spektrum zu 11-(2-Biphenylyl)-3,3-bis(4-methoxyphenyl)-5-brom-11-hydroxy-fluoreno[2,1-b]pyran umgesetzt. Dieses wird analog Beispiel 1 Schritt ix) cyclisiert zu Spiro-9-fluoren-11'-[3,3-bis(4-methoxyphenyl)-5-brom-fluoreno[2,1-b]pyran] (Spiro 12; 0.4 g), das mittels NMR-Spektrum identifiziert wurde.

Herstellung der Probekörper:

500 ppm des jeweiligen photochromen Farbstoffes werden im verwendeten Monomer (TRANSHADE-150 der Firma Tokuyama; Brechungsindex 1,52) bei Raumtemperatur unter Rühren gelöst. Nach Zugabe eines Initiators vom Alkylperoxyester-Typ (1,5 Gew.-%) wird zweimal entgast und anschließend nach dem von der Firma Tokuyama empfohlenen Temperaturprogramm polymerisiert. Die Glasgießformen sind schwarz gefärbt, damit die Gesamtheit der photochromen Farbstoffe im nichtangeregten Zustand in die Matrix eingebaut werden kann. Nach Beendigung der Polymerisation werden die Probekörper noch 2 h bei 100°C getempert.

Ermittlung der Kinetikwerte, a*/b*-Farborte sowie längstwelligen Absorptionsmaxima:

Zur Ermittlung der Aufhellgeschwindigkeiten wurden die hergestellten Probekörper in einer Kinetikbank PTM II der Fa. Zeiss vermessen (Bestrahlung mit 50 klux gemäß DIN EN 1836 Punkt 6.1.3.1.1.). Die Belichtungszeit beträgt jeweils 15 min, die Aufhellung im Dunkeln 10 min. Die Temperatur des Glases wird über eine thermostatisierbare Küvette geregelt. Während Belichtung und Aufhellung wird die Transmission - bewertet nach der Hellempfindlichkeit des menschlichen Auges V $\lambda$ - jeweils in kurzen Zeitintervallen aufgezeichnet. Darüber hinaus liefert die PTM II-Kinetikbank jeweils in kurzen Zeitintervallen die a*/b*-Farbort-Daten der Probekörper gemäß CIE 1976 sowie zum Belichtungsende das UV/VIS-Spektrum der eingedunkelten Probekörper, woraus die längstwelligen Absorptionsmaxima ermittelt werden.

Ermittlung des Gelbindex-Wertes vi sowie des Leistungserhaltes LE:

Der Gelbindex yi wird gemäß der Norm ASTM D 1925-70 ermittelt. Dazu werden die x/y/z-Farbort-Daten der Probekörper gemäß CIE 1931 mit der Normlichtart C ermittelt. Je größer der Betrag des Gelbindex-Wertes yi ist, umso größer ist die Vergilbung. Dieser Wert ist besonders aussagekräftig für Lebensdauertests der Probekörper. Dazu werden die Probekörper 50 h in einem Schnellbelichtungsgerät SUNTEST CPS der Fa. Heraeus (ausgestattet mit einem Xenon-Strahler sowie einem UV-Spezialglasfilter mit Cutoff 290 nm) belichtet. Der Unterschied der Gelbindex-Werte $\Delta$ yi vor bzw. nach diesem Xenontest ist ein Maß dafür, wie sehr der Probekörper im Lebensdauertest vergilbt. Der Leistungserhalt LE ist ein Maß dafür, wie tief der Probekörper nach dem Lebensdauertest noch eindunkelt. d.h. inwieweit die photochromen Farbstoffe noch reagieren.

## Patentansprüche

1. Photochrome Spirofluorenopyrane der Formel (I):

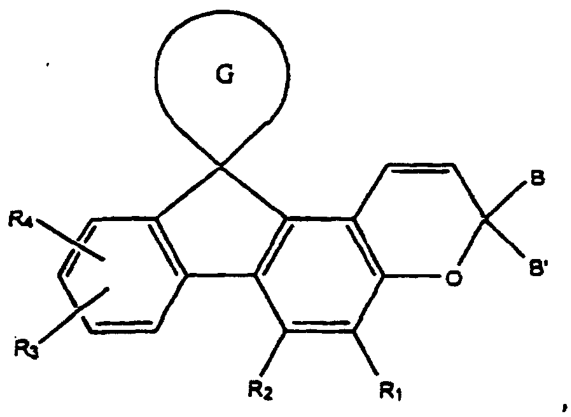

(I)

worin

| | |
|---|---|
| $R_1$ | ein Substituent, ausgewählt aus der Gruppe A, bestehend aus ($C_1$-$C_6$)-Alkyl, ($C_1$-$C_6$)-Alkoxy, Phenyl, Brom, Chlor und Fluor, ist; |
| $R_2$, $R_3$ und $R_4$ | unabhängig voneinander gleich oder verschieden sind und einen Substituenten, ausgewählt aus der Gruppe A', bestehend aus Wasserstoff und den Substituenten der Gruppe A, darstellen; oder |
| ($R_1$ | zusammen mit $R_2$) und/oder ($R_3$ zusammen mit $R_4$) unabhängig voneinander einen unsubstituierten, mono- oder disubstituierten Benzo- oder Pyridoring darstellen, dessen Substituenten aus |

der Gruppe A ausgewählt sind;

G unter Einrechnung des Spiro-Kohlenstoffatoms einen 5- bis 8-gliedrigen Ring darstellt, an den mindestens ein aromatisches oder heteroaromatisches Ringsystem annelliert ist, wobei das oder die Ringsysteme ausgewählt sind aus der Gruppe E, bestehend aus Benzol, Naphthalin, Phenanthren, Pyridin, Chinolin, Furan, Thiophen, Pyrrol, Benzofuran, Benzothiophen, Indol und Carbazol, die wiederum einen oder zwei Substituenten, ausgewählt aus der Gruppe A, aufweisen können;
und
B und B'
unabhängig voneinander aus den folgenden Gruppen a), b), c) oder d) ausgewählt sind, mit

a) Phenyl- oder Napthyl, die un-, mono-, di- oder trisubstituiert sind; oder
b) den Heterozyklen Pyridyl, Furanyl, Benzofuranyl, Thienyl, Benzothienyl und Julolidinyl, die un-, mono- oder disubstituiert sind,
wobei der oder die Substituenten der Aryl- oder Heteroarylgruppen in a) und b) aus einer Gruppe F, bestehend aus Hydroxy, Amino, Mono-($C_1$-$C_6$-alkylamino. Di-($C_1$-$C_6$)-alkylamino, am Phenylring un-, mono- oder disubstituiertem Mono- und Diphenylamino, Piperidinyl, N- substituiertem Piperazinyl, Pyrrolidinyl, Imidazolidinyl, Pyrazolidinyl, Indolinyl, Morpholinyl, Carbazolyl, un-, mono- und disubstituiertem Pyrryl, ($C_1$-$C_6$)-Alkyl, ($C_1$-$C_6$)-Alkoxy, Brom, Chlor und Fluor ausgewählt sind, wobei der oder die Substituenten an den Aromaten und Heteroaromaten aus einer Gruppe, bestehend aus ($C_1$-$C_6$)-Alkyl, ($C_1$-$C_6$)-Alkoxy, Brom, Chlor und Fluor, ausgewählt sind; oder
c) den Gruppen mit den folgenden Strukturbildern:

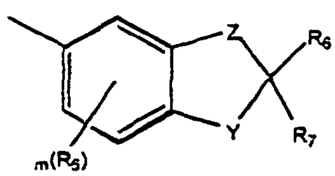
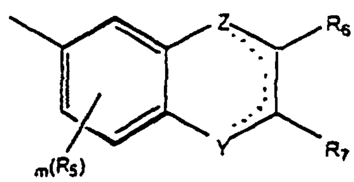
,

worin Y und Z unabhängig voneinander aus der Gruppe O, S, CH, $CH_2$, $NR^N$ und die Stickstoffsubstituenten $R^N$ aus der Gruppe, bestehend aus ($C_1$-$C_6$)-Alkyl, ($C_1$-$C_6$)-Acyl, Phenyl und Wasserstoff, ausgewählt sind und die Substituenten $R_5$ aus der Gruppe A und Hydroxy ausgewählt sind, wobei m = 0, 1 oder 2 ist, und $R_6$ und $R_7$ unabhängig voneinander Wasserstoff und/oder ($C_1$-$C_6$)-Alkyl sind; oder

d) B und B' zusammen un-, mono- oder disubstituiertes Fluoren-9-yliden oder einen gesättigten Kohlenwasserstoff darstellen, der $C_3$-$C_{12}$-spiro-monozyklisch, $C_7$-$C_{12}$-spiro-bizyklisch und/oder $C_7$-$C_{12}$-spiro-trizyklisch ist, wobei die Fluorensubstituenten aus der Gruppe A ausgewählt sind.

**2.** Photochrome Spirofluorenopyrane, insbesondere nach Anspruch 1, der Formel (II):

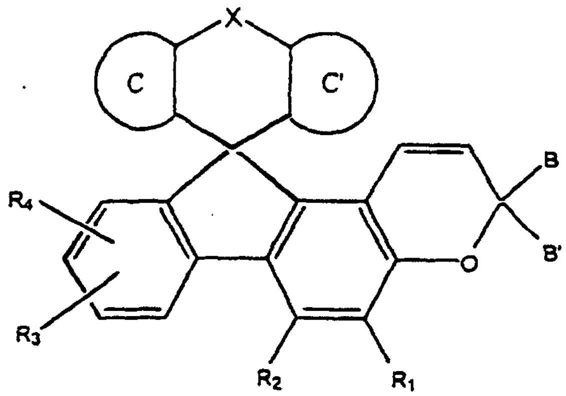

,

(II)

worin $R_1$ bis $R_4$, B und B' wie in Anspruch 1 definiert sind;

X entweder eine Einfachbindung ist, oder ausgewählt ist aus der Gruppe, bestehend aus

-O-, -S-, $-(CR_2)_n-$ mit n = 1, 2 oder 3,
-D=D'- mit D, D' = N, CR oder
-L-L'- mit L, L' = O, S, NR, CHR oder $CR_2$,

wobei R = H, $(C_1-C_6)$-Alkyl oder Phenyl und L und L' beide nicht gleichzeitig O oder S sein können;
und
C und C' unabhängig voneinander aus der in Anspruch 1 definierten Gruppe E ausgewählt sind, die jeweils einen oder zwei Substituenten, ausgewählt aus der Gruppe A aufweisen können.

**3.** Photochrome Spirofluorenopyrane nach Anspruch 2, **dadurch gekennzeichnet, daß** X eine Einfachbindung darstellt und die beiden Ringsysteme C und C' durch eine weitere Verknüpfung in ortho,ortho'-Stellung zur ersten Verknüpfung verbrückt sind.

**4.** Photochrome Spirofluorenopyrane nach Anspruch 3, **dadurch gekennzeichnet, daß** die weitere Verknüpfung in ortho,ortho'-Stellung mit den beiden Ringsystemen C und C' zu einer 4,5-Phenanthryl-Spiroverbindung führt.

**5.** Photochrome Spirofluorenopyrane nach mindestens einem der Ansprüche 1 bis 4, **dadurch gekennzeichnet, daß** B und B' unabhängig voneinander aus der Gruppe a) oder d) ausgewählt sind.

**6.** Photochrome Spirofluorenopyrane nach Anspruch 5, **dadurch gekennzeichnet, daß** B und B' unabhängig voneinander un- oder monosubstituiertes Phenyl oder Naphthyl darstellen, wobei der Substituent jeweils aus der Gruppe F ausgewählt ist.

**7.** Photochrome Spirofluorenopyrane nach mindestens einem der vorhergehenden Ansprüche, **dadurch gekennzeichnet, daß** B und B' die gleichen Substituenten darstellen.

**8.** Photochrome Spirofluorenopyrane nach mindestens einem der vorhergehenden Ansprüche, **dadurch gekennzeichnet, daß** C und C' unabhängig voneinander un- oder monosubstituiertes Phenyl oder Naphthyl darstellen, wobei der Substituent jeweils aus der Gruppe A ausgewählt ist.

**9.** Photochrome Spirofluorenopyrane nach mindestens einem der vorhergehenden Ansprüche, **dadurch gekennzeichnet, daß** C und C' die gleichen Substituenten darstellen.

**10.** Photochrome Spirofluorenopyrane, ausgewählt aus der Gruppe, bestehend aus:

1) Spiro-9-fluoren-13'-[3,3-diphenyl-6-methoxy-indeno[2,1-f]naphtho[1,2-b]pyran];
2) Spiro-9-fluoren-13'-[6-methoxy-3-(4-methoxyphenyl)-3-phenyl-indeno[2,1-f]-naphtho[1,2-b]pyran];
3) Spiro-9-xanthen-13'-[6-methoxy-3-(4-methoxyphenyl)-3-phenyl-indeno[2,1-f]-naphtho[1,2-b]yran];
4) Spiro-9-fluoren-13'-[3,3-bis(4-methoxyphenyl)-6-methoxy-indeno[2,1-f]naphtho-[1,2-b]pyran];
5) Spiro-9-xanthen-13'-[3,3-bis(4-methoxyphenyl)-6-methoxy-indeno[2,1-f]-naphtho[1,2-b]pyran];
6) Spiro-9-(9,10-dihydroanthracen)-13'-[3,3-bis(4-methoxyphenyl)-6-methoxyindeno[2,1-f]naphtho[1,2-b]pyran];
7) Spiro-9-fluoren-13'-{6-methoxy-3-[4-(N-morpholinyl)phenyl]-3-phenyl-indeno[2,1-f]-naphtho[1,2-b]pyran};
8) Spiro-9-fluoren-13'-[3-(4-dimethylaminophenyl)-6-methoxy-3-phenylindeno[2,1-f]-naphtho[1,2-b]pyran];
9) Spiro-9-fluoren-13'-[3-(4-diphenylaminophenyl)-6-methoxy-3-phenylindeno[2,1-f]-naphtho[1,2-b]pyran];
10) Spiro-9-fluoren-13'-[3,3-bis(4-methoxyphenyl)-indeno[2,1-f]naphtho[1,2-b]pyran];
11) Spiro-9-fluoren-13'-{3-[4-(N-morpholinyl)phenyl]-3-phenyl-indeno[2,1-f]naphtho-[1,2-b]pyran};
12) Spiro-9-fluoren-11'-[3,3-bis(4-methoxyphenyl)-3-brom-fluoreno[2,1-b]pyran] oder
13) Spiro-9-fluoren-13'-[3,3-bis(4-methoxyphenyl)-5-brom-benzo[1]fluoreno[2,1-b]pyran].

**11.** Verwendung eines oder mehrerer der photochromen Spirofluorenopyrane nach mindestens einem der Ansprüche 1 bis 10 in Kunststoffen aller Art, insbesondere für ophthalmische Zwecke.

**12.** Verwendung eines oder mehrerer der photochromen Spirofluorenopyrane nach mindestens einem der Ansprüche 1 bis 10 in Kunststoffgegenständen, insbesondere Linsen, Gläsern für Brillen aller Art, wie Skibrillen, Sonnenbrillen, Motorradbrillen, Schutzbrillen, Visieren für Schutzhelme, Fenstern, Schutzblenden, Abdeckungen, Dächern oder dergleichen.

## Claims

**1.** Photochromic spirofluorenopyrans of the formula (I):

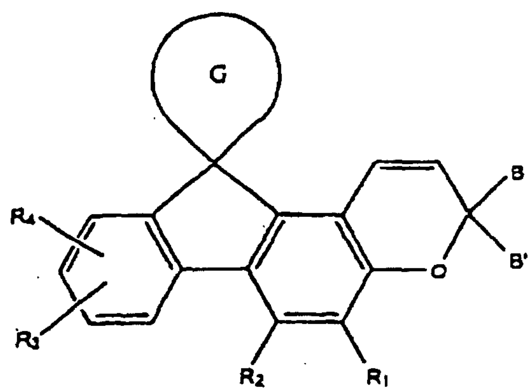

(I)

wherein

| | |
|---|---|
| $R_1$ | is a substituent selected from the group A which comprises $(C_1-C_6)$alkyl, $(C_1-C_6)$alkoxy, phenyl, bromine, chlorine and fluorine; |
| $R_2$, $R_3$ and $R_4$ | independently of each other are the same or different and represent a substituent selected from the group A' which comprises hydrogen and the substituents of the group A; or |
| ($R_1$ | together with $R_2$) and/or ($R_3$ together with $R_4$) independently of each other represent an unsubstituted, mono- or disubstituted benzo or pyrido ring, the substituents of which are selected from the group A; |
| G | represents, including the spirocarbon atom, a 5 to 8 membered ring, on which at least one aromatic or heteroaromatic ring system is anellated, the ring system(s) being selected from the |

group E which comprises benzene, naphthalene, phenanthrene, pyridine, quinoline, furan, thiophene, pyrrole, benzofuran,

benzothiophene, indole and carbazole, which in turn are able to have one or two substituents selected from the group A; and
B and B'
independently of each other are selected from the following groups a), b), c) or d), with

a) phenyl or naphthyl, which are un-, mono-, di- or trisubstituted;
or
b) the heterocycles pyridyl, furanyl, benzofuranyl, thienyl, benzothienyl and julolidinyl, which are un-, mono-, or disubstituted;
the substituent(s) of the aryl or heteroaryl groups in a) and b) being selected from a group F which comprises hydroxy, amino, mono-($C_1$-$C_6$)alkylamino, di-($C_1$-$C_6$)alkylamino, on the phenyl ring, un-, mono- or disubstituted mono- and diphenylamino, piperidinyl, N-substituted piperazinyl, pyrrolidinyl, imidazolidinyl, pyrazolidinyl, indolinyl, morpholinyl, carbazolyl, un-, mono- and disubstituted pyrryl, ($C_1$-$C_6$)alkyl, ($C_1$-$C_6$)alkoxy, bromine, chlorine and fluorine, the substituent(s) on the aromatics and heteroaromatics being selected from a group which comprises ($C_1$-$C_6$)alkyl, ($C_1$-$C_6$)alkoxy, bromine, chlorine and fluorine;
or
c) the groups with the following structural images;

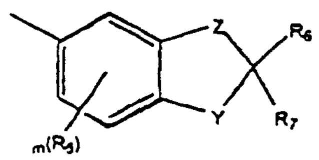

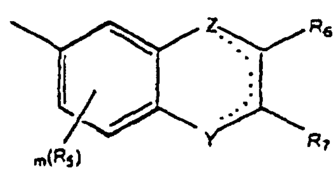

wherein Y and Z independently of each other are selected from the group O, S, CH, $CH_2$, $NR^N$ and the nitrogen substituents $R^N$ are selected from the group which comprises ($C_1$-$C_6$)alkyl, ($C_1$-$C_6$)acyl, phenyl and hydrogen, and
the substituents $R_5$ are selected from the group A and hydroxy, m being = 0, 1 or 2, and
$R_6$ and $R_7$ independently of each other are hydrogen and/or ($C_1$-$C_6$)alkyl;
or
d) B and B' together represent un-, mono- or disubstituted fluoren-9-ylides or a saturated hydrocarbon, which is $C_3$-$C_{12}$ spiromonocyclic, $C_7$-$C_{12}$ spiro-bicylic and/or $C_7$-$C_{12}$ spiro-tricyclic, the fluorene substituents being selected from the group A.

**2.** Photochromic spirofluorenopyrans according to claim 1, of the formula (II):

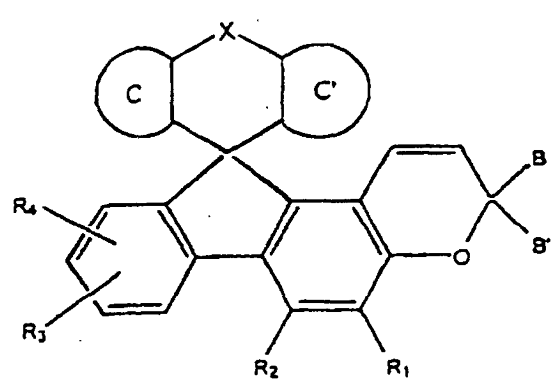

(II)

wherein $R_1$ to $R_4$, B and B' are defined as in claim 1;

X is either a single bond or is selected from the group which comprises

-O-, -S-, $-(CR_2)_n-$ with n = 1, 2 or 3,
-D=D'- with D, D' = N, CR or
-L-L'- with L, L' = O, S, NR, CHR or $CR_2$,
R being = H, $(C_1-C_6)$alkyl or phenyl and L and L' both being able, not at the same time, to be O or S;

and
C and C' independently of each other are selected from the group E defined in claim 1, which respectively can have one or two substituents selected from the group A.

**3.** Photochromic spirofluorenopyrans according to claim 2, **characterised in that** X represents a single bond and both ring systems C and C' are bridged by a further cross-linkage in ortho,ortho' position relative to the first cross-linkage.

**4.** Photochromic spirofluorenopyrans according to claim 3, **characterised in that** the further cross-linkage in ortho, ortho' position with the two ring systems C and C' leads to a 4,5-phenanthryl spirocompound.

**5.** Photochromic spirofluorenopyrans according to at least one of the claims 1 to 4, **characterised in that** B and B' independently of each other are selected from the group a) or d).

**6.** Photochromic spirofluorenopyrans according to claim 5, **characterised in that** B and B' independently of each other represent un- or monosubstituted phenyl or naphthyl, the substituent respectively being selected from the group F.

**7.** Photochromic spirofluorenopyrans according to at least one of the preceding claims, **characterised in that** B and B' represent the same substituents.

**8.** Photochromic spirofluorenopyrans according to at least one of the preceding claims, **characterised in that** C and C' independently of each other represent un- or monosubstituted phenyl or naphthyl, the substituent respectively being selected from the group A.

**9.** Photochromic spirofluorenopyrans according to at least one of the preceding claims, **characterised in that** C and C' represent the same substituents.

**10.** Photochromic spirofluorenopyrans selected from the group which comprises:

1) spiro-9-fluorene-13'-[3,3-diphenyl-6-methoxy-indeno[2,1-f]naphtho[1,2-b]pyran];
2) spiro-9-fluorene-13'-[6-methoxy-3-(4-methoxyphenyl)-3-phenyl-indeno[2,1-f]naphtho[1,2-b]pyran];
3) spiro-9-xanthene-13'-[6-methoxy-3-(4-methoxyphenyl)-3-phenyl-indeno[2,1-f]naphtho[1,2-b]pyran];
4) spiro-9-fluorene-13'-[3,3-bis(4-methoxyphenyl)-6-methoxy-indeno[2,1-f]naphtho-[1,2-b]pyran];
5) spiro-9-xanthene-13'-[3,3-bis(4-methoxyphenyl)-6-methoxy-indeno[2,1-f]naphtho[1,2-b]pyran];
6) spiro-9-(9,10-dihydroanthracene)-13'-[3,3-bis(4-methoxyphenyl)-6-methoxy-indeno[2,1-f]naphtho[1,2-b]pyran];
7) spiro-9-fluorene-13'-{6-methoxy-3-[4-(N-morpholinyl)phenyl]-3-phenyl-indeno[2,1-f]-naphtho[1,2-b]pyran};
8) spiro-9-fluorene-13'-[3-4-dimethylaminophenyl)-6-methoxy-3-phenyl-indeno[2,1-f]-naphtho[1,2-b]pyran];
9) spiro-9-fluorene-13'-[3-(4-diphenylaminophenyl)-6-methoxy-3-phenyl-indeno[2,1-f]-naphtho[1,2-b]pyran];
10) spiro-9-fluorene-13'-[3,3-bis(4-methoxyphenyl)-indeno[2,1-f]naphtho[1,2-b]pyran];
11) spiro-9-fluorene-13'-{3-[4-(N-morpholinyl)phenyl]-3-phenyl-indeno[2,1-f]naphtho-[1,2-b]pyran];
12) spiro-9-fluorene-11'-[3,3-bis(4-methoxyphenyl)-5-bromine-fluoreno[2,1-b]pyran]
or
13) spiro-9-fluorene-13'-[3,3-bis(4-methoxyphenyl)-5-bromine-benzo[1]fluoreno[2,1-b]pyran].

**11.** Use of one or more of the photochromic spirofluorenopyrans according to at least one of the claims 1 to 10 in plastic materials of all types, in particular for ophthalmic purposes.

**12.** Use of one or more of the photochromic spirofluorenopyrans according to at least one of the claims 1 to 10 in plastic material objects, in particular lenses, glasses for spectacles of all types, such as ski goggles, sunglasses, motorcycle goggles, protective goggles, visors for protective helmets, windows, protective screens, coverings, roofs or the like.

## Revendications

**1.** Spirofluorénopyranne photochrome de la formule (I) :

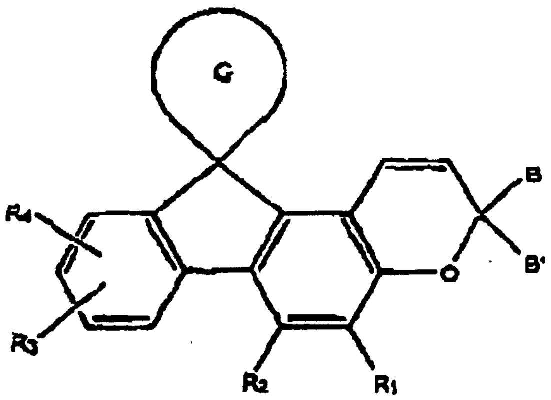

(I)

où

$R_1$ est un substituant choisi parmi le groupe A, consistant en alkyle ($C_1$-$C_6$), alcoxy ($C_1$-$C_6$), phényle, brome, chlore ou fluor ;

$R_2$, $R_3$ et $R_4$ sont indépendamment l'un de l'autre, identiques ou différents et représentent un substituant choisi parmi le groupe A', consistant en hydrogène et les substituants du groupe A, ou

($R_1$ avec $R_2$) et/ou ($R_3$ avec $R_4$) représentent indépendamment l'un de l'autre, un cycle benzène ou pyridine non substitué, mono- ou disubstitué, dont les substituants sont choisis parmi le groupe A ;

G représente en incluant l'atome de carbone spiro, un cycle ayant 5 à 8 membres, sur lequel est condensé au moins un cycle aromatique ou hétéroaromatique, où le ou les systèmes cycliques sont choisis parmi le groupe E, consistant en le benzène, le naphtalène, le phénathrène, la pyridine, la quinoléine, le furanne, le thiophène, le pyrrole, le benzofuranne, le benzothiophène, l'indole et le carbazole, qui peuvent à nouveau présenter un ou deux substituants choisis parmi la groupe A, et

B et B' sont choisis indépendamment l'un de l'autre, parmi les groupes a), b), c) ou d) suivants, avec

a) phényle ou naphtyle, qui sont non, mono-, di- ou trisubstitués ;

b) les hétérocycles pyridyle, furannyle, benzofurannyle, thiényle, benzothiényle et julodinyle, qui sont non, mono- ou disubstitués ;

où le ou les substituants des radicaux aryle et hétéroaryle dans a) et b) sont choisis parmi le groupe F, consistant en hydroxyle, amino, monoalkyle ($C_1$-$C_6$)-amino, dialkyle ($C_1$-$C_6$)-amino, mono- ou diphénylamino, non, mono- ou disubstitué sur le cycle phényle, pipéridinyle, pipérazinyle N-substitué, pyrrolidinyle, imidazolidinyle, pyrazolidinyle, indolinyle, morpholinyle, carbazolyle, pyrryle non, mono- et disubstitué, alkyle ($C_1$-$C_6$), alcoxy ($C_1$-$C_6$), brome, chlore et fluor, où le ou les substituants sur les aromatiques et hétéroaromatiques sont choisis parmi un groupe consistant en alkyle ($C_1$-$C_6$), alcoxy ($C_1$-$C_6$), brome, chlore et fluor ; ou

c) les groupements avec les structures suivantes :

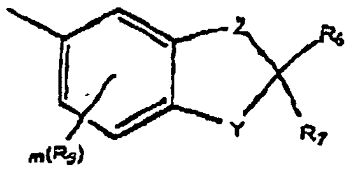 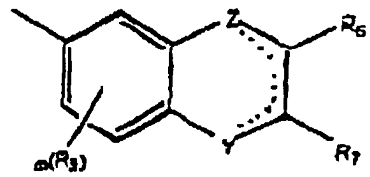 .

où Y et Z sont choisis indépendamment l'un de l'autre, parmi le groupe O, S, CH, $CH_2$, $NR^N$ et les substituants de l'azote $R^N$ sont choisis parmi le groupe consistant en alkyle ($C_1$-$C_6$), acyle ($C_1$-$C_6$), phényle et hydrogène, et

les substituants $R_5$ sont choisis parmi le groupe A et hydroxyle, où m = 0, 1 ou 2, et

$R_6$ et $R_7$ sont indépendamment l'un de l'autre, hydrogène et/ou alkyle ($C_1$-$C_6$), ou

d) B et B' représentent ensemble, un fluorèn-9-ylidène non, mono- ou disubstitué ou un hydrocarbure saturé, qui est spiro-monocyclique en $C_3$-$C_{12}$, spiro-bcyclique en $C_7$-$C_{12}$ et/ou spiro-tricyclique en $C_7$-$C_{12}$, où les substituants du fluorène sont choisis parmi le groupe A.

2. Spirofluorénopyranne photochrome, en particulier selon la revendication 1, de la formule (II) :

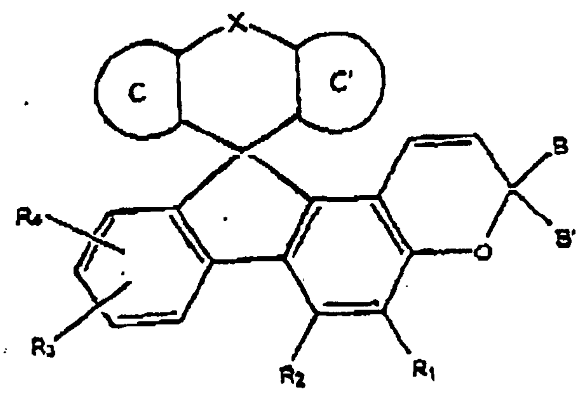 ,

où $R_1$ à $R_4$, B et B' sont définis comme à la revendication 1 ;
X est une simple liaison ou est choisi parmi le groupe consistant en :

-O-, -S-, -($CR_2$)n-, avec n = 1, 2 ou 3 ;
-D=D'-, avec D, D' = N, CR, ou
-L-L'-, avec L, L' = O, S, NR, CHR ou $CR_2$,

où R = H, alkyle ($C_1$-$C_6$), phényle et L et L' ne peuvent pas être simultanément O ou S ;
et
C et C' sont choisis indépendamment l'un de l'autre, parmi le groupe E défini à la revendication 1, qui peuvent présenter chaque fois, un ou deux substituants choisis parmi le groupe A.

3. Spirofluorénopyranne photochrome selon la revendication 2, **caractérisé en ce que** X représente une simple liaison et que les deux systèmes cycliques C et C' sont pontés par une autre jonction en position ortho,ortho' à la première jonction.

4. Spirofluorénopyranne photochrome selon la revendication 3, **caractérisé en ce que** l'autre jonction en position ortho,ortho' avec les deux systèmes cycliques C et C' conduit à un composé spiro 4,5-phénanthryle.

5. Spirofluorénopyranne photochrome selon au moins l'un des revendications 1 à 4, **caractérisé en ce que** B et B' sont choisis indépendamment l'un de l'autre, parmi le groupe a) ou d).

6. Spirofluorénopyranne photochrome selon la revendication 5, **caractérisé en ce que** B et B' représentent indépendamment l'un de l'autre, un phényle
ou naphtyle non ou monosubstitué, où le substituant est chaque fois choisi parmi le groupe F.

7. Spirofluorénopyranne photochrome selon au moins l'un des revendications précédentes, **caractérisé en ce que** B et B' représentent les mêmes substituants.

8. Spirofluorénopyranne photochrome selon au moins l'un des revendications précédentes, **caractérisé en ce que** C et C' représentent indépendamment l'un de l'autre, un phényle ou naphtyle non ou monosubstitué, où le substituant est chaque fois choisi parmi le groupe A.

9. Spirofluorénopyranne photochrome selon au moins l'un des revendications précédentes, **caractérisé en ce que** C et C' représentent les mêmes substituants.

10. Spirofluorénopyranne photochrome, choisi parmi le groupe consistant en :

1) le spiro-9-fluorène-13'-[3,3-diphényl-6-méthoxy-indéno-[2,1-f]-naphto[1,2-b]pyranne] ;
2) le spiro-9-fluorène-13'-[6-méthoxy-3-(4-méthoxy-phényl)-3-phénylindéno[2,1-f]naphto[1,2-b]pyranne] ;
3) le spiro-9-xanthène-13'-[6-méthoxy-3-(4-méthoxy-phényl)-3-phénylindéno[2,1-f]naphto[1,2-b]pyranne] ;
4) le spiro-9-fluorène13'-[3,3-bis(4-méthoxyphényl)-6-méthoxyindéno[2,1-f]naphto[1,2-b]pyranne] ;
5) le spiro-9-xanthène-13'-[3,3-bis(4-méthoxyphényl)-6-méthoxy-indéno[2,1-f]naphto[1,2-b]pyranne] ;
6) le spiro-9-(9,10-dihydroanthracène)-13'-[3,3-bis (4-méthoxyphényl) -6-méthoxyindéno[2,1-f]naphto[1, 2-b] pyranne] ;
7) le spiro-9-fluorène-13'-[6-méthoxy-3-(4-N-morpholinyl-phényl)-3-phénylindéno[2,1-f]naphto[1,2-b] pyranne] ;
8) le spiro-9-fluorène-13'-[3-(4-diméthylaminophényl)-6-méthoxy-3-phénylindéno[2,1-f]naphto[1,2-b] pyranne] ;
9) le spiro-9-fluorène-13'-[3-(4-diphénylaminophényl) -6-méthoxy-3-phénylindéno[2, 1-f]naphto[1,2-b] pyranne] ;
10) le spiro-9-fluorène-13'-[3,3-bis(4-méthoxyphényl ) -indéno[2,1-f]naphto[1,2-b]pyranne] ;
11) le spiro-9-fluorène-13'-[3-(4-N-morpholinyl-phényl)-3-phénylindéno[2,1-f]naphto[1,2-b]pyranne] ;
12) le spiro-9-fluorène-11'-[3,3-bis(4-méthoxyphényl)-5-bromofluoréno[2,1-b]pyranne], ou
13) le spiro-9-fluorène-13'-[3,3-bis(4-méthoxyphényl)-5-bromobenzofluoréno[2,1-b]pyranne].

11. Utilisation d'un ou de plusieurs spirofluorénopyrannes photochromes selon au moins une des revendications 1 à 10 dans des matières plastiques de tout type, en particulier pour des objets ophtalmiques.

12. Utilisation d'un ou de plusieurs spirofluorénopyrannes photochromes selon au moins une des revendications 1 à 10 dans des objets en plastique, en particulier des lentilles, des verres pour lunettes de tout type, comme des lunettes de ski, des lunettes solaires, des lunettes pour motard, des lunettes de protection, des visières pour casque de protection, des vitres, des écrans de protection, des couvercles, des toits ou similaires.

Figur 1

Kinetikdaten im Vergleich (23 °C, 15 min Belichtung
bei 50 klux, 10 min Dunkelaufhellung

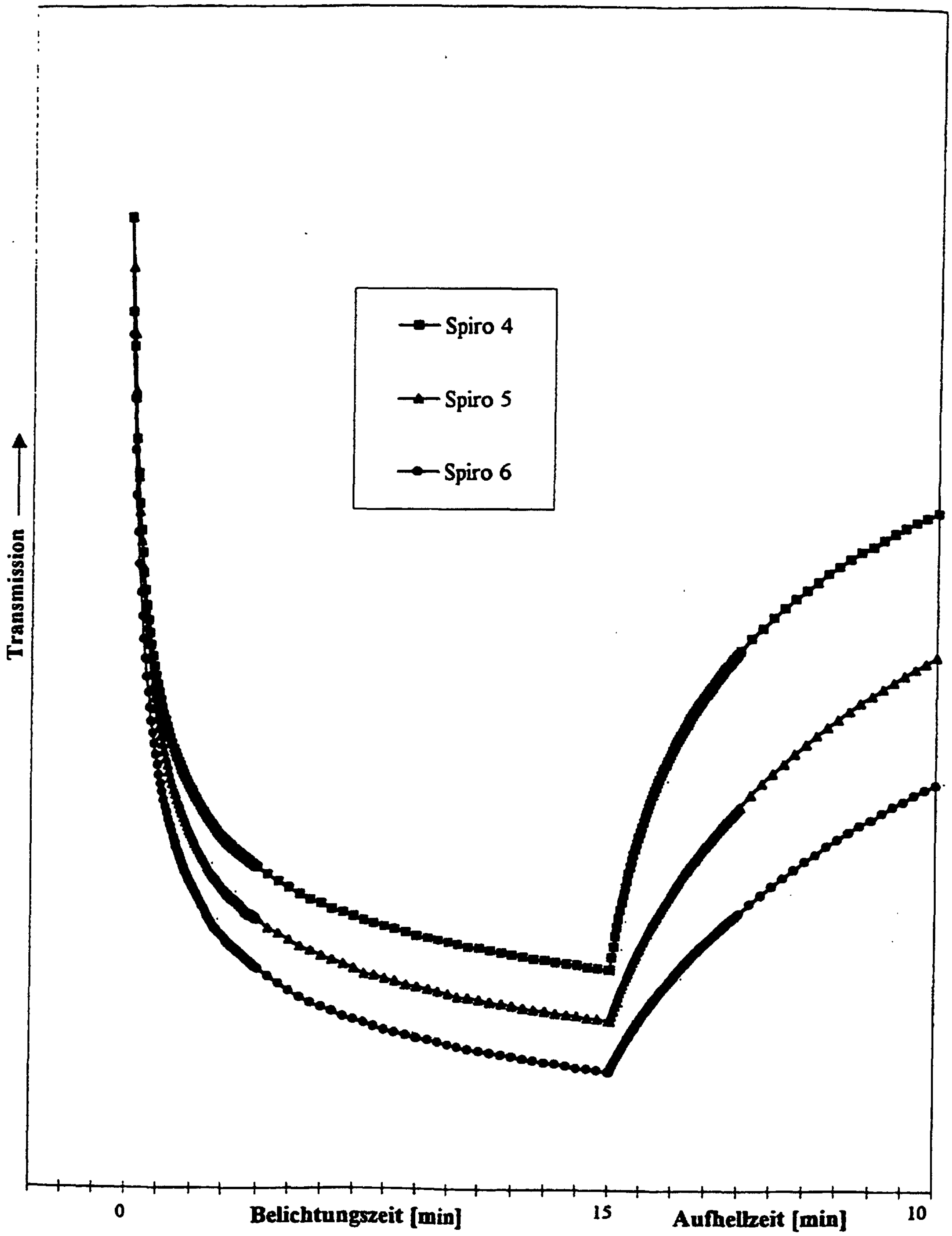

Figur 2

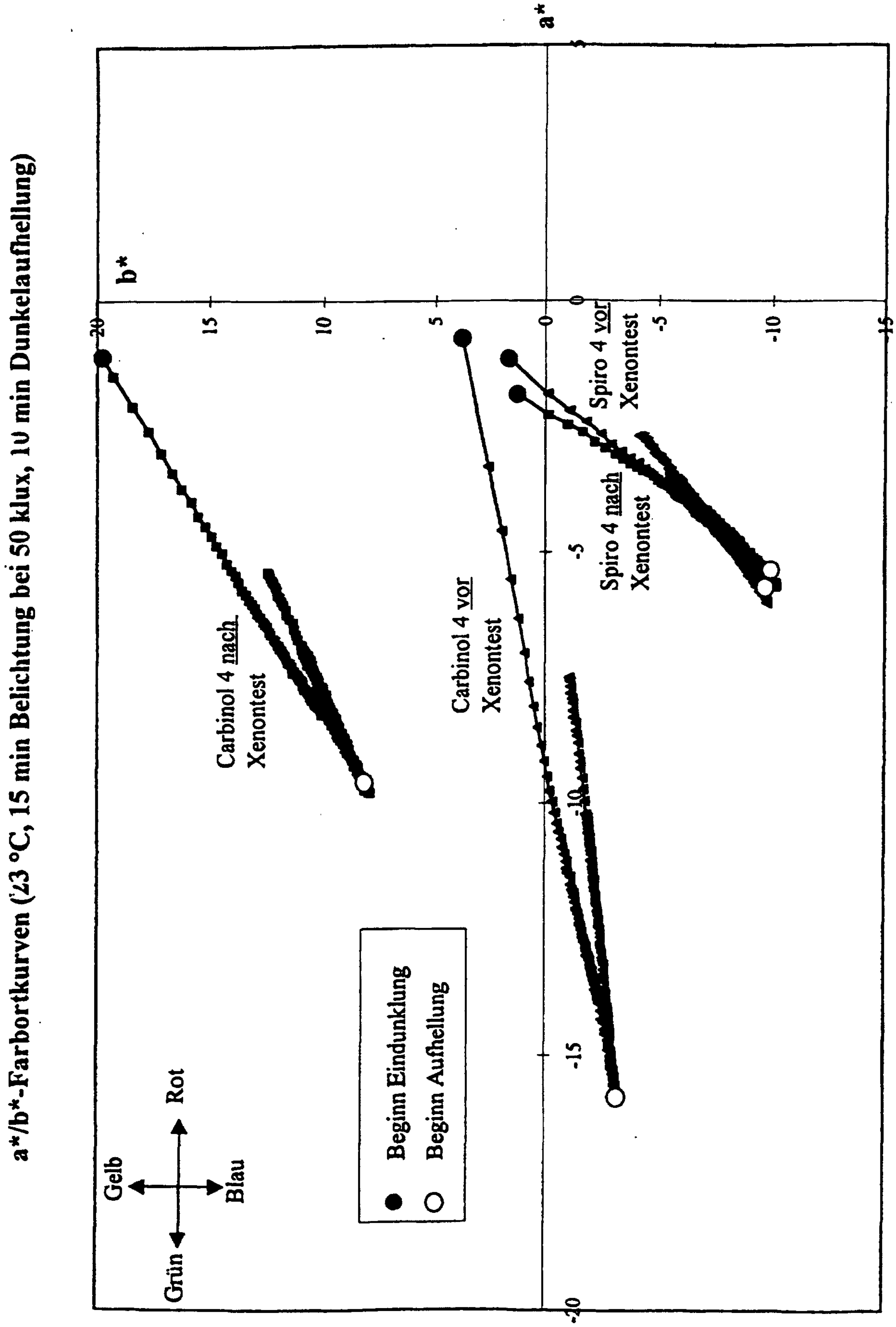
a*/b*-Farbortkurven (23 °C, 15 min Belichtung bei 50 klux, 10 min Dunkelaufhellung)
a*
b*
20
15
10
5
0
-5
-10
-15
-20
Carbinol 4 nach Xenontest
Carbinol 4 vor Xenontest
Spiro 4 vor Xenontest
Spiro 4 nach Xenontest
Gelb
Rot
Grün
Blau
Beginn Eindunklung
Beginn Aufhellung

Figur 3

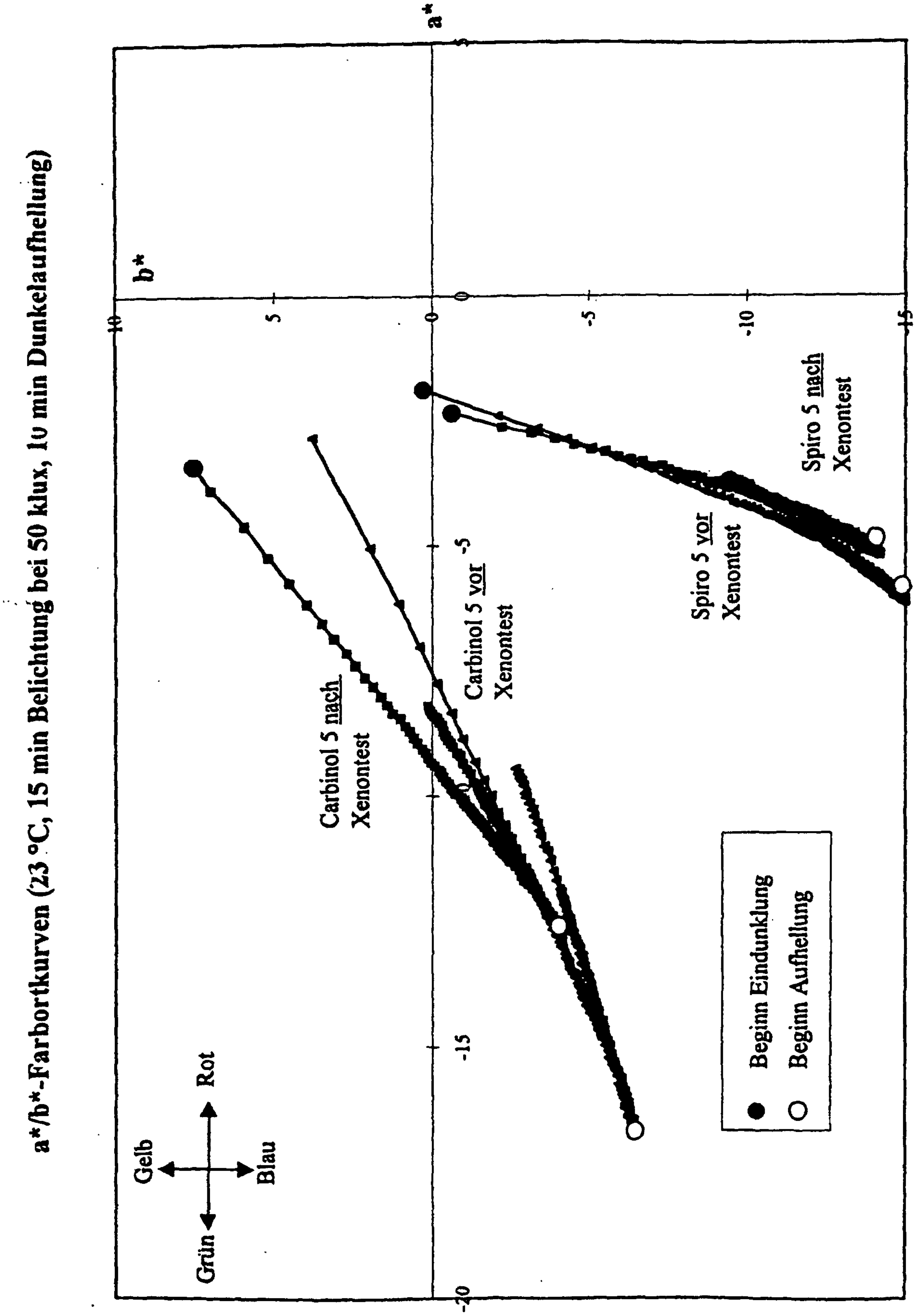
a*/b*-Farbortkurven (23 °C, 15 min Belichtung bei 50 klux, 10 min Dunkelaufhellung)
a*
b*
10
5
0
-5
-10
-15
-20
Gelb
Rot
Blau
Grün
Carbinol 5 nach Xenontest
Carbinol 5 vor Xenontest
Spiro 5 vor Xenontest
Spiro 5 nach Xenontest
Beginn Eindunklung
Beginn Aufhellung

Figur 4

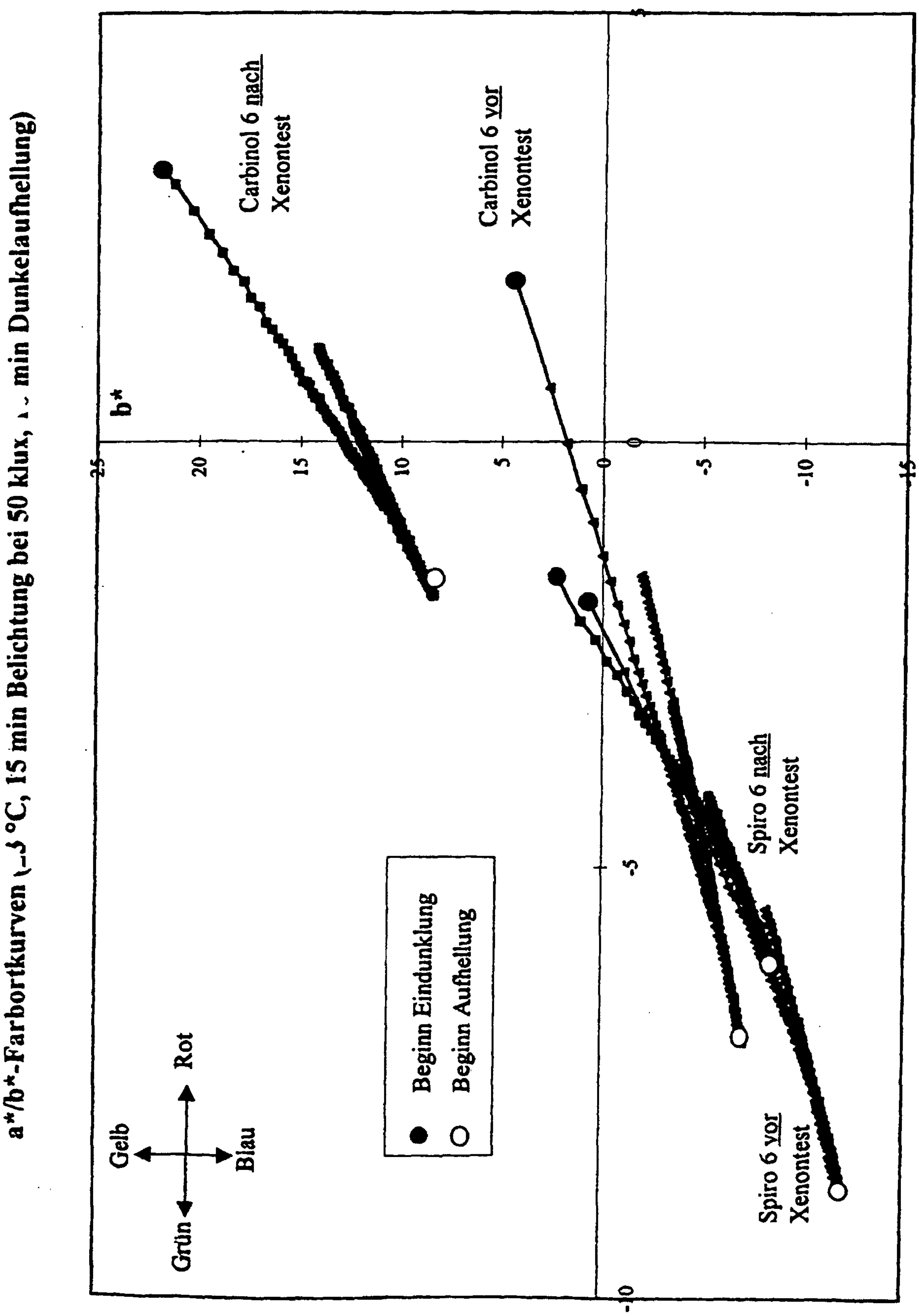
a*/b*-Farbortkurven ( °C, 15 min Belichtung bei 50 klux, min Dunkelaufhellung)
a*
b*
25
20
15
10
5
0
-5
-10
-15
-10
-5
Carbinol 6 nach Xenontest
Carbinol 6 vor Xenontest
Spiro 6 nach Xenontest
Spiro 6 vor Xenontest
Gelb
Rot
Blau
Grün
Beginn Eindunklung
Beginn Aufhellung

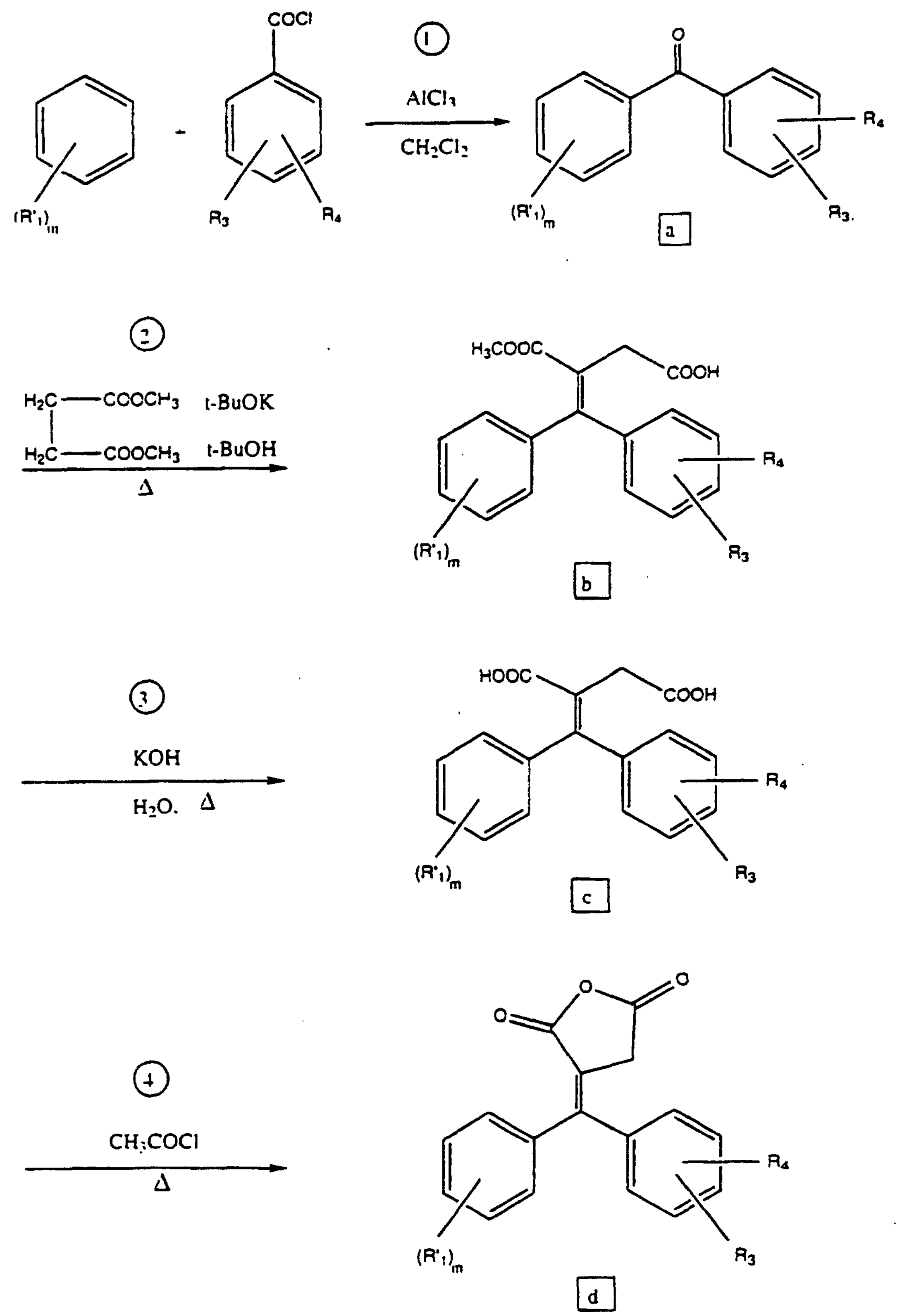


Fig. 5

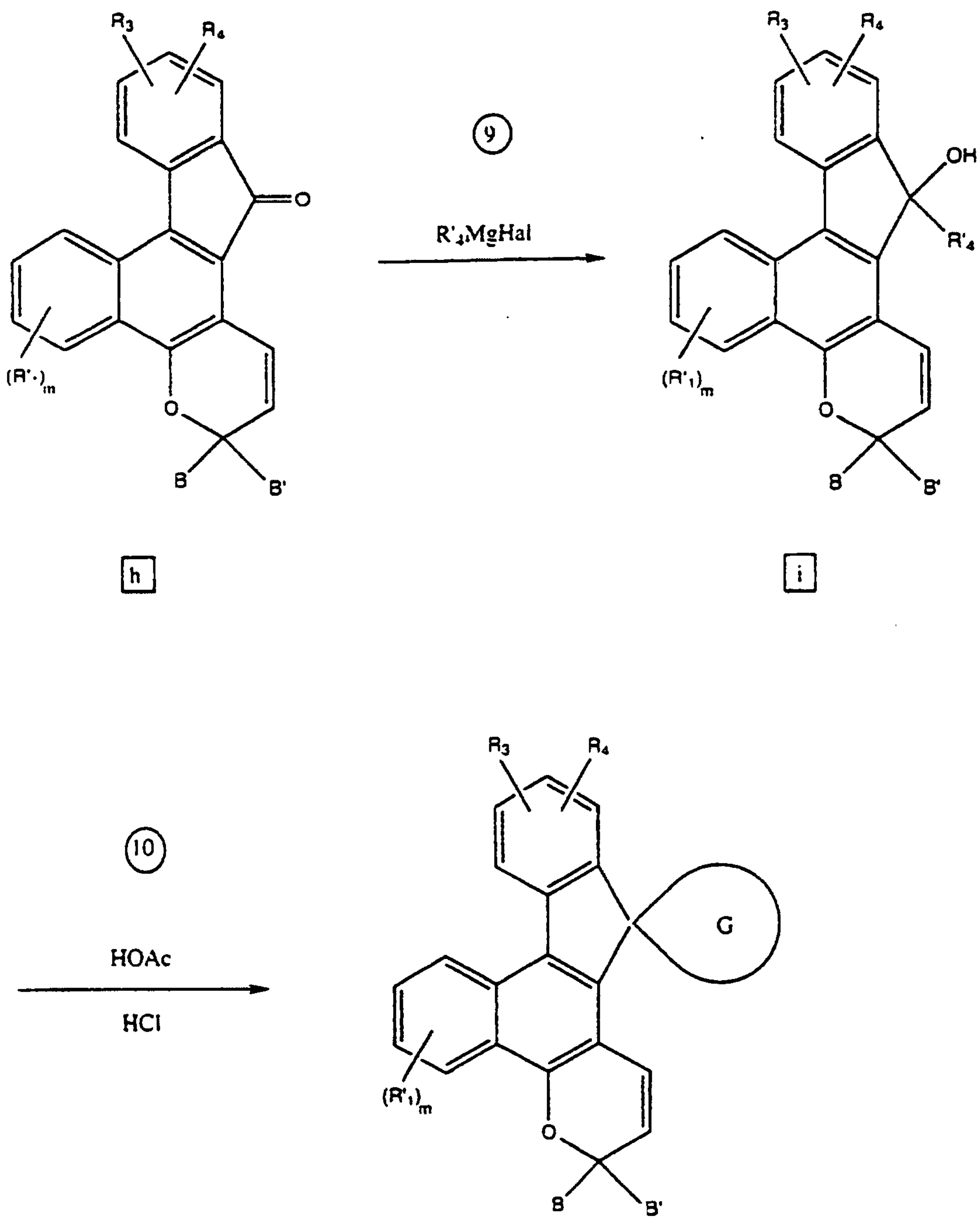


h

i

k

Fig. 6